# EUROPEAN PATENT APPLICATION

(11) **EP 4 834 356 A1**
(43) Date of publication of application: **04.11.2026**
(21) Application number: 24911316.8
(22) Date of filing: 26.12.2024
(51) Int. Cl.: A61K 36/605, A61K 31/445, A61P 19/10, A61P 19/02, A61P 21/00

(54) **USE OF MULBERRY EXTRACT IN PREPARING DRUG FOR TREATING AND/OR PREVENTING ABNORMAL BONE METABOLISM-RELATED DISEASES**

(30) Priority: 26.12.2023 CN 202311810068; 25.12.2024 CN 202411923461
(71) Applicant: Beijing Wehand-Bio Pharmaceutical Co., Ltd, Beijing 102600 (CN); Guangxi Wehand-Bio Pharmaceutical Co., Ltd, Hechi, Guangxi 546300 (CN)
(72) Inventor: LIU, Yuling, Beijing 102600 (CN); CHEN, Yanmin, Beijing 102600 (CN); LIU, Zhihua, Beijing 102600 (CN); ZHU, Xiangyang, Beijing 102600 (CN); JIN, Yiqun, Beijing 102600 (CN); LIU, Dongdong, Beijing 102600 (CN); FAN, Kuangshi, Beijing 102600 (CN); LIU, Yuanyuan, Beijing 102600 (CN); LI, Huijuan, Beijing 102600 (CN); LU, Wenchao, Beijing 102600 (CN); FENG, Yu, Beijing 102600 (CN); ZHANG, Mengya, Beijing 102600 (CN); WANG, Tingting, Beijing 102600 (CN); ZOU, Yuanyuan, Beijing 102600 (CN); CAO, Haiyan, Beijing 102600 (CN)
(74) Representative: Bandpay & Greuter
(86) International application number: PCT/CN2024/142684
(87) International publication number: WO 2025/140394

(57) **Abstract**

Use of mulberry extract in preparing a drug for treating and/or preventing abnormal bone metabolism-related diseases. The mulberry extract comprises: based on the sum of the weight percentages of components in the mulberry extract being 100%: 3% to 99% of alkaloids, 0.2% to 70% of polysaccharides, 0% to 10% of flavonoidss, 0% to 50% of amino acids, and 0% to 25% of other components.

## Description

### Technical Field

The present disclosure relates to the field of bio-medicine, and particularly relates to the use of a mulberry extract in preparing a drug for treating and/or preventing diseases related to abnormal bone metabolism.

### Background

Bone is a tissue that continuously performs remodeling metabolic activities, which consists of two opposite processes: bone formation (by osteoblasts) and bone resorption (by osteoclasts). Under normal circumstances, bone resorption and bone formation are closely related to various hormones (such as PTH, vitamin D, steroids, calcitonin, etc.) and intrinsic regulatory factors (such as cytokines, growth factors, etc.). Bone resorption is regulated by RANKL and M-CSF, and is inhibited by OPG. Bone formation is induced by a variety of biological factors, and meanwhile is modulated by M-CSF, ALP, osteocalcin, osteopontin and osteonectin. The imbalance between bone resorption and bone formation leads to metabolic bone diseases, such as osteoporosis, osteoarthritis, rheumatoid arthritis, bone dislocation and so on.

Osteoporosis (OP) is a common metabolic bone disease characterized by reduced bone mass and destruction of bone tissue micro-structure, which results in increased bone fragility, decreased bone strength and elevated fracture risks. The stability of bone metabolism is regulated by the balance between bone formation induced by osteoblasts and bone resorption induced by osteoclasts. Primary osteoporosis is the most common clinical type, accounting for approximately 80% of all cases. It can be classified into Type I high-turnover osteoporosis, which is predominantly characterized by increased bone resorption and commonly seen in postmenopausal women, and Type II low-turnover osteoporosis, which is predominantly characterized by decreased bone formation and is commonly seen in the elderly.

Sarcopenia is a clinical syndrome characterized by progressive and generalized loss of muscle content and muscle strength, which increases the risks of physical disability, declined quality of life, and mortality. Sarcopenia and osteoporosis are closely correlated degenerative diseases, and are mainly manifested by the interaction between muscle and bone. Declined muscle content can induce and accelerate the occurrence and progression of osteoporosis, while decreased bone strength also promotes muscle atrophy and function deterioration.

At present, there have been no reports on the use of mulberry extract for treating diseases related to abnormal bone metabolism.

### Summary

The objective of the present disclosure is to provide use of a mulberry extract in preparing a drug for treating and/or preventing diseases related to abnormal bone metabolism. The mulberry extract of the present disclosure exhibits significantly favorable therapeutic effects on diseases related to abnormal bone metabolism.

In the present disclosure, taking total weight percentage content of all components in the mulberry extract as 100%, the mulberry extract contains: alkaloids with a weight content of no less than 3% (optionally contains alkaloids with a weight content ranging from 3% to 99%, further optionally contains alkaloids with a weight content ranging from 15% to 99%, further optionally contains alkaloids with a weight content ranging from 30% to 99%, further optionally contains alkaloids with a weight content ranging from 40% to 99%, further optionally contains alkaloids with a weight content ranging from 50% to 99%, further optionally contains alkaloids with a weight content ranging from 60% to 99%, further optionally contains alkaloids with a weight content ranging from 35% to 70%, and further optionally contains alkaloids with a weight content ranging from 60% to 75%);
and/or polysaccharides with a weight content of no more than 70% (optionally contains polysaccharides with a weight content ranging from 0.2% to 70%, further optionally contains polysaccharides with a weight content ranging from 0.2% to 50%, further optionally contains polysaccharides with a weight content ranging from 0.2% to 35%, further optionally contains polysaccharides with a weight content ranging from 0.2% to 25%, further optionally contains polysaccharides with a weight content ranging from 0.2% to 23%, and further optionally contains polysaccharides with a weight content ranging from 20% to 25%);
and/or flavonoids with a weight content of no more than 10% (optionally contains flavonoids with a weight content ranging from 0.05% to 5%, further optionally contains flavonoids with a weight content ranging from 0% to 2%, further optionally contains flavonoids with a weight content ranging from 0.05% to 2%, further optionally contains flavonoids with a weight content ranging from 0.5% to 1.5%, further optionally contains flavonoids with a weight content ranging from 0% to 1%, and further optionally contains flavonoids with a weight content ranging from 0.05% to 1%);
and/or amino acids with a weight content of no more than 50% (optionally contains amino acids with a weight content ranging from 0% to 30%, further optionally contains amino acids with a weight content ranging from 0% to 25%, further optionally contains amino acids with a weight content ranging from 0% to 20%, further optionally contains amino acids with a weight content ranging from 0% to 5%, further optionally contains amino acids with a weight content ranging from 3% to 25%, and further optionally contains amino acids with a weight content ranging from 5% to 20%);
and/or other components (optionally with a weight content ranging from 0% to 25%, further optionally with a weight content ranging from 0% to 20%, further optionally with a weight content ranging from 0% to 15%, further optionally with a weight content ranging from 0% to 11%, further optionally with a weight content ranging from 2% to 20%, and further optionally with a weight content ranging from 4% to 8%).

Optionally, the diseases related to abnormal bone metabolism are any one of osteoporosis, sarcopenia, sarcopenia-osteoporosis, muscular atrophy, myasthenia, osteomalacia, rickets, primary hyperparathyroid osteopathy, fluorosis of bone, osteoarthritis and bone dislocation; optionally, the disease related to abnormal bone metabolism is steoporosis, sarcopenia or sarcopenia-osteoporosis.

Besides, the diseases related to abnormal bone metabolism may also be diseases causing reduced bone mineral density such as nephropathy, liver disease, diabetes and rheumatoid arthritis.

Optionally, the use of the mulberry extract in preparing a drug for treating and/or preventing diseases related to abnormal bone metabolism includes any one of uses described in the following (b1) to (b3):
(b1) use in preparing a drug for increasing bone mineral density;
(b2) use in preparing a drug for reducing bone marrow lipid vacuoles; and
(b3) use in preparing a drug for improving bone micro-structure;

The improvement of bone micro-structure includes at least one of the following: (i) increasing trabecular number; (ii) reducing trabecular separation; (iii) reducing trabecular structure model index; and (iv) increasing bone volume fraction;
optionally, the use of the mulberry extract in preparing a drug for treating and/or preventing diseases related to abnormal bone metabolism includes any one of the following:
1) use in preparing a drug for increasing muscle content;
2) use in preparing a drug for increasing muscle weight percentage;
3) use in preparing a drug for increasing muscle-fat ratio;
4) use in preparing a drug for inhibiting muscle atrophy and/or edema; optionally, the inhibition of muscle atrophy and/or edema is specifically to increase cross-sectional area of muscle fibers, muscle fiber diameter and/or muscle fiber perimeter;
5) use in preparing a drug for alleviating intermuscular peripheral neuropathy and/or vascular lipid deposition;
6) use in preparing a drug for inhibiting muscle cell injury; and
7) use in preparing a drug for increasing muscle strength.

In an optional embodiment of the present disclosure, taking total weight percentage content of all components in the mulberry extract as 100%, the weight content of each component in the mulberry extract is as follows:

| | |
|---|---|
| alkaloids | 3% to 99%, |
| polysaccharides | 0.2% to 70%, |
| flavonoids | 0% to 10%, |
| amino acids | 0% to 50%, and |
| other components | 0% to 25%; |

optionally, taking total weight percentage content of all components in the mulberry extract as 100%, the weight content of each component in the mulberry extract is as follows:

| | |
|---|---|
| alkaloids | 30% to 99%, |
| polysaccharides | 0.2% to 35%, |
| flavonoids | 0% to 2%, |
| amino acids | 0% to 30%, and |
| other components | 0% to 20%; |

optionally, taking total weight percentage content of all components in the mulberry extract as 100%, the weight content of each component in the mulberry extract is as follows:

| | |
|---|---|
| alkaloids | 30% to 99%, |
| polysaccharides | 0.2% to 35%, |
| flavonoids | 0.05% to 2%, |
| amino acids | 0% to 25%, and |
| other components | 0% to 20%; |

optionally, taking total weight percentage content of all components in the mulberry extract as 100%, the weight content of each component in the mulberry extract is as follows:

| | |
|---|---|
| alkaloids | 40% to 99%, |
| polysaccharides | 0.2% to 25%, |
| flavonoids | 0.05% to 1%, |
| amino acids | 0% to 25%, and |
| other components | 0% to 15%; |

optionally, taking total weight percentage content of all components in the mulberry extract as 100%, the weight content of each component in the mulberry extract is as follows:

| | |
|---|---|
| alkaloids | 50% to 99%, |
| polysaccharides | 0.2% to 23%, |
| flavonoids | 0% to 1%, |
| amino acids | 0% to 20%, and |
| other components | 0% to 11%; |

optionally, taking total weight percentage content of all components in the mulberry extract as 100%, the weight content of each component in the mulberry extract is as follows:

| | |
|---|---|
| alkaloids | 50% to 99%, |
| polysaccharides | 0.2% to 35%, |
| flavonoids | 0% to 2%, |
| amino acids | 0% to 30%, and |
| other components | 0% to 20%; |

optionally, taking total weight percentage content of all components in the mulberry extract as 100%, the weight content of each component in the mulberry extract is as follows:

| | |
|---|---|
| alkaloids | 60% to 99%, |
| polysaccharides | 0.2% to 23%, |
| flavonoids | 0% to 1%, |
| amino acids | 0% to 5%, and |
| other components | 0% to 11%; and |

further optionally, taking total weight percentage content of all components in the mulberry extract as 100%, the weight content of each component in the mulberry extract is as follows:

| | |
|---|---|
| alkaloids | 50% to 65%, |
| polysaccharides | 20% to 25%, |
| flavonoids | 0.5% to 1.5%, |
| amino acids | 3% to 25%, and |
| other components | 2% to 20%. |

Further optionally, taking total weight percentage content of all components in the mulberry extract as 100%, the weight content of each component in the mulberry extract is as follows:

| | |
|---|---|
| alkaloids | 50% to 65%, |
| polysaccharides | 20% to 25%, |
| flavonoids | 0.5% to 1.5%, |
| amino acids | 5% to 20%, and |
| other components | 4% to 8%. |

Further optionally, taking total weight percentage content of all components in the mulberry extract as 100%, the weight content of each component in the mulberry extract is as follows:

| | |
|---|---|
| alkaloids | 40% to 65%, |
| polysaccharides | 22% to 25%, |
| flavonoids | 0.5% to 1%, |
| amino acids | 5% to 25%, and |
| other components | 5% to 10%; |

optionally, taking total weight percentage content of all components in the mulberry extract as 100%, the weight content of each component in the mulberry extract is as follows:

| | |
|---|---|
| alkaloids | 30% to 90%, |
| polysaccharides | 0.2% to 35%, |
| flavonoids | 0% to 2%, |
| amino acids | 0% to 30%, and |
| other components | 0% to 20%; |

optionally, taking total weight percentage content of all components in the mulberry extract as 100%, the weight content of each component in the mulberry extract is as follows:

| | |
|---|---|
| alkaloids | 30% to 90%, |
| polysaccharides | 0.2% to 35%, |
| flavonoids | 0.05% to 2%, |
| amino acids | 0% to 25%, and |
| other components | 0% to 20%; |

optionally, taking total weight percentage content of all components in the mulberry extract as 100%, the weight content of each component in the mulberry extract is as follows:

| | |
|---|---|
| alkaloids | 30% to 80%, |
| polysaccharides | 0.2% to 35%, |
| flavonoids | 0% to 2%, |
| amino acids | 0% to 30%, and |
| other components | 0% to 11%; |

optionally, taking total weight percentage content of all components in the mulberry extract as 100%, the weight content of each component in the mulberry extract is as follows:

| | |
|---|---|
| alkaloids | 30% to 80%, |
| polysaccharides | 5% to 35%, |
| flavonoids | 0.1% to 2%, |
| amino acids | 4% to 25%, and |
| other components | 8% to 11%; |

optionally, taking total weight percentage content of all components in the mulberry extract as 100%, the weight content of each component in the mulberry extract is as follows:

| | |
|---|---|
| alkaloids | 30% to 70%, |
| polysaccharides | 0.2% to 35%, |
| flavonoids | 0% to 2%, |
| amino acids | 0% to 25%, and |
| other components | 0% to 8%; |

optionally, taking total weight percentage content of all components in the mulberry extract as 100%, the weight content of each component in the mulberry extract is as follows:

| | |
|---|---|
| alkaloids | 30% to 70%, |
| polysaccharides | 20% to 35%, |
| flavonoids | 0.6% to 2%, |
| amino acids | 5% to 25%, and |
| other components | 4% to 8%; |

optionally, taking total weight percentage content of all components in the mulberry extract as 100%, the weight content of each component in the mulberry extract is as follows:

| | |
|---|---|
| alkaloids | 35% to 70%, |
| polysaccharides | 0.2% to 35%, |
| flavonoids | 0% to 2%, |
| amino acids | 0% to 25%, and |
| other components | 0% to 8%; |

optionally, taking total weight percentage content of all components in the mulberry extract as 100%, the weight content of each component in the mulberry extract is as follows:

| | |
|---|---|
| alkaloids | 35% to 70%, |
| polysaccharides | 20% to 35%, |
| flavonoids | 0.6% to 2%, |
| amino acids | 5% to 25%, and |
| other components | 4% to 8%; |

optionally, taking total weight percentage content of all components in the mulberry extract as 100%, the weight content of each component in the mulberry extract is as follows:

| | |
|---|---|
| alkaloids | 40% to 70%, |
| polysaccharides | 0.2% to 25%, |
| flavonoids | 0.05% to 1%, |
| amino acids | 0% to 25%, and |
| other components | 0% to 10%; |

optionally, taking total weight percentage content of all components in the mulberry extract as 100%, the weight content of each component in the mulberry extract is as follows:

| | |
|---|---|
| alkaloids | 40% to 70%, |
| polysaccharides | 20% to 25%, |
| flavonoids | 0.05% to 1%, |
| amino acids | 5% to 25%, and |
| other components | 0% to 10%; |

optionally, taking total weight percentage content of all components in the mulberry extract as 100%, the weight content of each component in the mulberry extract is as follows:

| | |
|---|---|
| alkaloids | 30% to 65%, |
| polysaccharides | 0.2% to 35%, |
| flavonoids | 0% to 2%, |
| amino acids | 0% to 25%, and |
| other components | 0% to 8%; |

optionally, taking total weight percentage content of all components in the mulberry extract as 100%, the weight content of each component in the mulberry extract is as follows:

| | |
|---|---|
| alkaloids | 30% to 65%, |
| polysaccharides | 20% to 35%, |
| flavonoids | 0.6% to 2%, |
| amino acids | 5% to 25%, and |
| other components | 4% to 8%; |

optionally, taking total weight percentage content of all components in the mulberry extract as 100%, the weight content of each component in the mulberry extract is as follows:

| | |
|---|---|
| alkaloids | 15% to 99%, |
| polysaccharides | 0.2% to 20%, |
| flavonoids | 0% to 10%, |
| amino acids | 0% to 45%, and |
| other components | 0% to 15%; |

optionally, taking total weight percentage content of all components in the mulberry extract as 100%, the weight content of each component in the mulberry extract is as follows:

| | |
|---|---|
| alkaloids | 15% to 90%, |
| polysaccharides | 3% to 20%, |
| flavonoids | 0% to 10%, |
| amino acids | 0% to 45%, and |
| other components | 0% to 15%; |

optionally, taking total weight percentage content of all components in the mulberry extract as 100%, the weight content of each component in the mulberry extract is as follows:

| | |
|---|---|
| alkaloids | 15% to 80%, |
| polysaccharides | 5% to 35%, |
| flavonoids | 0% to 10%, |
| amino acids | 0% to 45%, and |
| other components | 0% to 15%; |

optionally, taking total weight percentage content of all components in the mulberry extract as 100%, the weight content of each component in the mulberry extract is as follows:

| | |
|---|---|
| alkaloids | 15% to 70%, |
| polysaccharides | 0.2% to 20%, |
| flavonoids | 0% to 10%, |
| amino acids | 0% to 45%, and |
| other components | 0% to 15%; |

optionally, taking total weight percentage content of all components in the mulberry extract as 100%, the weight content of each component in the mulberry extract is as follows:

| | |
|---|---|
| alkaloids | 15% to 70%, |
| polysaccharides | 0.2% to 20%, |
| flavonoids | 0% to 10%, |
| amino acids | 5% to 45%, and |
| other components | 0% to 15%; |

optionally, taking total weight percentage content of all components in the mulberry extract as 100%, the weight content of each component in the mulberry extract is as follows:

| | |
|---|---|
| alkaloids | 15% to 65%, |
| polysaccharides | 0.2% to 20%, |
| flavonoids | 0% to 10%, |
| amino acids | 0% to 45%, and |
| other components | 0% to 15%; |

optionally, taking total weight percentage content of all components in the mulberry extract as 100%, the weight content of each component in the mulberry extract is as follows:

| | |
|---|---|
| alkaloids | 15% to 65%, |
| polysaccharides | 0.2% to 20%, |
| flavonoids | 0% to 10%, |
| amino acids | 5% to 45%, and |
| other components | 0% to 15%; |

optionally, taking total weight percentage content of all components in the mulberry extract as 100%, the weight content of each component in the mulberry extract is as follows:

| | |
|---|---|
| alkaloids | 15% to 30%, |
| polysaccharides | 0.2% to 40%, |
| flavonoids | 0% to 10%, |
| amino acids | 20% to 50%, and |
| other components | 0% to 15%; |

optionally, taking total weight percentage content of all components in the mulberry extract as 100%, the weight content of each component in the mulberry extract is as follows:

| | |
|---|---|
| alkaloids | 3% to 70%, |
| polysaccharides | 0.2% to 70%, |
| flavonoids | 0% to 10%, |
| amino acids | 0% to 10%, and |
| other components | 0% to 10%; |

optionally, taking total weight percentage content of all components in the mulberry extract as 100%, the weight content of each component in the mulberry extract is as follows:

| | |
|---|---|
| alkaloids | 3% to 70%, |
| polysaccharides | 20% to 70%, |
| flavonoids | 0% to 10%, |
| amino acids | 5% to 10%, and |
| other components | 0% to 10%; |

optionally, taking total weight percentage content of all components in the mulberry extract as 100%, the weight content of each component in the mulberry extract is as follows:

| | |
|---|---|
| alkaloids | 3% to 30%, |
| polysaccharides | 35% to 70%, |
| flavonoids | 0% to 10%, |
| amino acids | 0% to 25%, and |
| other components | 0% to 10%; |

optionally, taking total weight percentage content of all components in the mulberry extract as 100%, the weight content of each component in the mulberry extract is as follows:

| | |
|---|---|
| alkaloids | 3% to 30%, |
| polysaccharides | 35% to 70%, |
| flavonoids | 0% to 10%, |
| amino acids | 0% to 25%, and |
| other components | 0% to 10%; |

optionally, taking total weight percentage content of all components in the mulberry extract as 100%, the weight content of each component in the mulberry extract is as follows:

| | |
|---|---|
| alkaloids | 3% to 40%, |
| polysaccharides | 25% to 70%, |
| flavonoids | 0% to 10%, |
| amino acids | 0% to 25%, and |
| other components | 0% to 10%; |

optionally, taking total weight percentage content of all components in the mulberry extract as 100%, the weight content of each component in the mulberry extract is as follows:

| | |
|---|---|
| alkaloids | 3% to 40%, |
| polysaccharides | 25% to 70%, |
| flavonoids | 0% to 10%, |
| amino acids | 10% to 25%, and |
| other components | 0% to 10%; |

optionally, taking total weight percentage content of all components in the mulberry extract as 100%, the weight content of each component in the mulberry extract is as follows:

| | |
|---|---|
| alkaloids | 3% to 50%, |
| polysaccharides | 20% to 70%, |
| flavonoids | 0% to 10%, |
| amino acids | 0% to 20%, and |
| other components | 0% to 10%; |

optionally, taking total weight percentage content of all components in the mulberry extract as 100%, the weight content of each component in the mulberry extract is as follows:

| | |
|---|---|
| alkaloids | 3% to 50%, |
| polysaccharides | 20% to 70%, |
| flavonoids | 0% to 10%, |
| amino acids | 10% to 20%, and |
| other components | 0% to 10%; |

optionally, taking total weight percentage content of all components in the mulberry extract as 100%, the weight content of each component in the mulberry extract is as follows:

| | |
|---|---|
| alkaloids | 3% to 65%, |
| polysaccharides | 20% to 70%, |
| flavonoids | 0% to 10%, |
| amino acids | 0% to 10%, and |
| other components | 0% to 10%; |

optionally, taking total weight percentage content of all components in the mulberry extract as 100%, the weight content of each component in the mulberry extract is as follows:

| | |
|---|---|
| alkaloids | 3% to 65%, |
| polysaccharides | 20% to 70%, |
| flavonoids | 0% to 10%, |
| amino acids | 5% to 10%, and |
| other components | 0% to 10%; |

optionally, taking total weight percentage content of all components in the mulberry extract as 100%, the weight content of each component in the mulberry extract is as follows:

| | |
|---|---|
| alkaloids | 80% to 99%, |
| polysaccharides | 0.2% to 10%, |
| flavonoids | 0% to 1%, |
| amino acids | 0% to 5%, and |
| other components | 0% to 15%; and |

the weight content of each component may be any of the above-mentioned value combinations.

Optionally, the alkaloids include one or more of 1-deoxynojirimycin (DNJ), N-methyl-1-deoxynojirimycin, fagomine (FAG), 3-epi-fagomine, 1,4-dideoxy-1,4-imino-D-arabinitol (DAB), calysteginB2, calysteginC1, 2-O-(α-D-galactopyranosyl)-1-deoxynojirimycin, 6-O-(β-D-glucopyranosyl)-1-deoxynojirimycin, and 1,4-dideoxy-1,4-imino-(2-O-β-D-glucopyranosyl)-D-arabinitol.

Optionally, weight percentage of DNJ is no less than 50% (optionally 60% to 99%) of total alkaloids.

Optionally, content of heavy metal in the mulberry extract is no more than 10 ppm.

The drug is in an oral dosage form; optionally, the drug is a tablet, a capsule, an oral solution, an oral emulsion, a pill, a granule, a syrup and a powder.

In the present disclosure, the mulberry extract may be provided in the form of commercially available Ramulus Mori Alkaloids Tablets (National Medicine Approval No. Z20200002).

In another aspect, the present disclosure provides a method for preventing and/or treating diseases related to abnormal bone metabolism, including administering to a patient in need thereof a therapeutically and/or prophylactically effective amount of the mulberry extract.

Optionally, the method for preventing and/or treating diseases related to abnormal bone metabolism includes administering to a patient in need thereof a therapeutically and/or prophylactically effective amount of the mulberry extract to improve any one of the following (b1) to (b3):
(b1) increasing bone mineral density;
(b2) reducing bone marrow lipid vacuoles; and
(b3) improving bone micro-structure; wherein:
the improvement of bone micro-structure includes at least one of the following: (i) increasing trabecular number; (ii) reducing trabecular separation; (iii) reducing trabecular structure model index; and (iv) increasing bone volume fraction.

Optionally, the method for preventing and/or treating diseases related to abnormal bone metabolism includes administering to a patient in need thereof a therapeutically and/or prophylactically effective amount of the mulberry extract to improve any one of the following 1) to 7):
1) increasing muscle content;
2) increasing muscle weight percentage;
3) increasing muscle-fat ratio;
4) inhibiting muscle atrophy and/or edema; optionally, the inhibition of muscle atrophy and/or edema is specifically to increase cross-sectional area of muscle fibers, muscle fiber diameter and/or muscle fiber perimeter;
5) alleviating intermuscular peripheral neuropathy and/or vascular lipid deposition;
6) inhibiting muscle cell injury; and
7) increasing muscle strength.

In another aspect, the present disclosure provides use of the mulberry extract in treating and/or preventing diseases related to abnormal bone metabolism.

Optionally, the use of the mulberry extract in treating and/or preventing diseases related to abnormal bone metabolism includes any one of the uses in the following (b1) to (b3):
(b1) use in increasing bone mineral density;
(b2) use in reducing bone marrow lipid vacuoles; and
(b3) use in improving bone micro-structure; wherein:
   improvements of bone micro-structure include at least one of the following: (i) increasing trabecular number; (ii) reducing trabecular separation; (iii) reducing trabecular structure model index; and (iv) increasing bone volume fraction;
   optionally, the use of the mulberry extract in treating and/or preventing diseases related to abnormal bone metabolism includes any one of the following:
      1) use in increasing muscle content;
      2) use in increasing muscle weight percentage;
      3) use in increasing muscle-fat ratio;
      4) use in inhibiting muscle atrophy and/or edema; optionally, the inhibition of muscle atrophy and/or edema is specifically to increase cross-sectional area of muscle fibers, muscle fiber diameter and/or muscle fiber perimeter;
      5) use in alleviating intermuscular peripheral neuropathy and/or vascular lipid deposition;
      6) use in inhibiting muscle cell injury; and
      7) use in increasing muscle strength.

Preferably, the bone includes tibia and femur.

The muscle includes skeletal muscle, such as gastrocnemius, tibialis anterior, quadriceps femoris and soleus. The mulberry extract acts on human beings or mammals.

Optionally, the treatment and/or prevention of diseases related to abnormal bone metabolism is reflected in improving any one of the patient's bone mass, bone quality, muscle content, muscle strength and physical balance; optionally, the patient suffers from any one of osteoporosis, sarcopenia, sarcopenia-osteoporosis (osteosarcopenia syndrome), muscular atrophy, myasthenia, osteomalacia, rickets, primary hyperparathyroid osteopathy, fluorosis of bone, osteoarthritis and bone dislocation; further optionally, the patient suffers from osteoporosis, sarcopenia or sarcopenia-osteoporosis.

Optionally, the diseases related to abnormal bone metabolism are caused by aging, endocrine disorder, obesity and/or diabetes.

Optionally, the mulberry extract of the present disclosure may be prepared according to the method disclosed in CN110393738A. In one embodiment, the preparation of the mulberry extract includes the following steps of: preparing a crude extraction solution of Moraceae plants; optionally, performing separation via a cation resin and/or an anion resin; optionally, subjecting resin effluent to alcohol precipitation and collecting the supernatant; and optionally, concentrating and/or drying the supernatant.

Optionally, the preparation of the mulberry extract in the present disclosure includes the following steps of: 1) preparing a crude extraction solution of Moraceae plants; 2) separating the crude extraction solution via a cation resin and/or an optional anion resin to obtain a resin effluent; optional step 3): subjecting the resin effluent afforded in step 2) to alcohol precipitation and collecting the supernatant; and 4) concentrating and/or drying the supernatant. Optionally, the resin effluent afforded in step 2) is concentrated and/or dried before alcohol precipitation.

Optionally, the Moraceae plants are one or a combination of several of Morus multicaulis Perrott., Morus alba L., Morus atropurpurea Roxb., Morus mizuho Hotta, Morus wittiorum Hand Mazz., Morus laevigata Wall, Morus nigra Linn., Morus cathayana Hemsi., Morus serrata Roxb., Morus mongolica Schneid., Morus bombycis Koidz., Morus notabilis Schneid., Morus nigriformis Koidz., Morus yunnanensis Koidz., Morus australis Poir., Morus mongolica (Bur.) Schneid var. diabolica Koidz., large-leaf mulberry, Morus alba Var. Pendula Dippel, white-vein mulberry, mulberry varieties bred from the above mulberry species, and intraspecific or interspecific hybrid mulberry selected from the above mulberry species.

Optionally, the Moraceae plants are selected from one or more of Morus atropurpurea Roxb., Morus multicaulis Perrott., Morus alba L., Morus serrata Roxb., Morus bombycis Koidz. and hybrid mulberry; wherein the hybrid mulberry is optionally Yuesang No.11, Morus atropurpurea cv. Guisangyou 62 or Morus atropurpurea cv. Sangteyou 2. All parts of the Moraceae plants can be used, including leaves, roots, branches, barks, buds, stems and fruits; optionally Ramulus Mories, mulberry leaves or mulberry bark can be used.

In the present disclosure, the mulberry extract may be Ramulus Mori extract, mulberry bark extract, mulberry leaf extract or a mixed extract thereof.

In one embodiment, the mulberry extract was prepared according to the following steps of: crushing Moraceae plants; heating for reflux extraction with water and/or alcohol solution or acidic water, the solvent dosage was 3 to 20 times of the raw medicinal material; extracting repeatedly for 1 to 3 times; combining and concentrating the extraction solutions; loading a cation exchange resin; eluting with 0.2 to 3 N ammonia water, loading the eluate onto an anion exchange resin, and collecting the non-adsorbed fraction; and adding ethanol to precipitate and remove impurities, and concentrating and/or drying, thereby affording the extract.

In one embodiment, the mulberry extract was prepared according to the following steps of: crushing Moraceae plants; heating for reflux extraction with water and/or alcohol solution or acidic water, wherein the solvent dosage was 3 to 20 times of the raw medicinal material; extracting repeatedly for 1 to 3 times; combining and concentrating the extraction solutions; loading a cation exchange resin; eluting with 0.2 to 3 N ammonia water, loading the eluate onto an anion exchange resin, and collecting the non-adsorbed fraction; and concentrating and/or drying, thereby affording the extract.

In one embodiment, the mulberry extract was prepared according to the following steps of: crushing Moraceae plants; heating for reflux extraction with water and/or alcohol solution or acidic water, wherein the solvent dosage was 3 to 20 times of the raw medicinal material; extracting repeatedly for 1 to 3 times; combining and concentrating the extraction solutions; loading a cation exchange resin; eluting with 0.2 to 3 N ammonia water, and concentrating and/or drying the eluate, thereby affording the extract.

In one embodiment, the mulberry extract was prepared according to the following steps of: crushing Moraceae plants; heating for reflux extraction with water, wherein the solvent dosage was 3 to 20 times of the raw medicinal material (optionally 4 to 15 times, further optionally 4 to 12 times); extracting repeatedly for 1 to 3 times (the extraction duration being optionally 0.5 to 3 h for each extraction, further optionally 1 to 2 h for each extraction); combining and concentrating the extraction solutions; loading a cation exchange resin; eluting with 0.2 to 3 N ammonia water, loading the eluate onto an anion exchange resin, and collecting the non-adsorbed fraction (i.e., anion resin effluent); and adding ethanol to precipitate and remove impurities, and concentrating and/or drying, thereby affording the extract.

Optionally, the concentrated crude extraction solution might be subjected to alcohol precipitation before resin separation in step 2). During alcohol precipitation, the crude extraction solution was added with ethanol, and then was stirred till the mixture was even; then stirring was stopped and the mixture was allowed to stand for a certain period of time so that insoluble substances therein precipitated. Optionally, counted in L/kg, the volume-mass ratio of added ethanol to plant raw material is 0.2 to 20 times, optionally 0.4 to 10 times. Further optionally, an alcohol precipitation tank was used for alcohol precipitation. Optionally, the stirring speed during alcohol precipitation was 10 to 600 rpm, optionally 40 to 500 rpm, further optionally 80 to 400 rpm or 300 rpm.

Optionally, after being packed into a column, the cation resin was activated by being washed with an acid solution, an alkaline solution, and an acid solution successively. Optionally, washing with an alkaline solution stopped until the pH of eluate reached 8.0 to 9.5, optionally 8.5 to 9.5; optionally, the alkaline solution was selected from ammonia solution, sodium hydroxide solution, potassium hydroxide solution or sodium carbonate solution; optionally, the concentration of the alkaline solution was 0.5 to 4 mol/L, optionally 1 to 2 mol/L. Optionally, the washing with an acid solution stopped until the pH of eluate reached 3.0 to 7.0, optionally 4.5 to 6.5. Optionally, the acid solution was selected from hydrochloric acid solution, phosphoric acid solution, disodium hydrogen phosphate-citric buffer; optionally, the concentration of the acid solution was 0.5 to 4 mol/L, optionally 1.5 to 2 mol/L. Optionally, after the last washing with an acid solution, the cation resin was rinsed with deionized water having a volume of 3 to 5 times the column volume.

Optionally, the cation resin is one or a combination of more selected from 732-type strongly acidic styrene-based cation exchange resin, 002SC-type strongly acidic styrene-based cation resin, 734-type strongly acidic styrene-based cation exchange resin, D001-type macroporous strongly acidic styrene-based cation exchange resin, D113-type macroporous and weakly acidic cation resin and D254-type macroporous and strongly alkaline quaternary ammonium-type cation exchange resin.

Optionally, the amount of the cation resin used and the charged plant raw material had a weight ratio ranging from 1:1 to 1:30 (optionally 1:1 to 1:25, 1:2 to 1:20, 1:2 to 1:15, 1:2 to 1:10, 1:2 to 1:7, 1:2 to 1:3).

After loading the plant crude extraction solution onto the cation resin, the loaded cation resin was eluted with an eluent. Optionally, the eluent is a salt solution or an alkaline solution containing cations, optionally one or more selected from sodium chloride, ammonium chloride, ammonium sulfate, ammonium nitrate, ammonia water, potassium chloride and sodium hydroxide.

Optionally, the eluent had a concentration ranging from 0.04 to 5 mol/L (optionally 0.5 to 2.5 mol/L, 0.2 to 3 mol/L, and further optionally 0.5 to 2.5 mol/L).

Optionally, the eluent had a flow rate ranging from 1 to 15 BV/h (optionally 5 to 10 BV/h, and further optionally 5 to 6 BV/h).

Optionally, the weight of the eluent used for the separation via cation resin was 0.1 to 30 times the weight of the charged plant raw material, optionally, the elution is perfromed with the eluent, of which the weight was 0.5 to 10 times the weight of the charged plant raw material.

The starting point of collection may be determined according to the pH of the cation resin effluent; for example, when an alkaline solution such as ammonia water is used for elution, the eluate was collected when the pH of the cation column effluent was detected to be higher than 7, or the collection starting point of the effluent was determined according to a chromogenic reaction or a precipitation reaction; optionally, the collection was terminated when the volume of the collection solution reached 0.1 to 10 times the weight of the charged plant raw material (further optionally 0.2 to 5 times), and the collection solution was optionally purified by passing through an anion column.

For purification via anion column, optionally, after being packed into the column, the anion resin was activated by being washed with an alkaline solution, an acid solution and an alkaline solution successively.

Optionally, the anion resin was washed with an alkaline solution until the pH of the eluate reached 8.0 to 9.5, optionally 8.5 to 9.5;

Optionally, the alkaline solution was selected from ammonia solution, sodium hydroxide solution, potassium hydroxide solution or sodium carbonate solution; optionally, the concentration of the alkaline solution was 0.5 to 4 mol/L, optionally 1 to 2 mol/L.

Optionally, the anion resin was washed with an acid solution until the pH of the eluate reached 3.0 to 7.0, optionally 4.5 to 6.5. Optionally, the acid solution was selected from hydrochloric acid solution, phosphoric acid solution and disodium hydrogen phosphate-citric buffer; optionally, the concentration of the acid solution is 0.5 to 4 mol/L, optionally 1 to 2 mol/L.

Optionally, the anion resin was one or a combination of more selected from 711-type strongly alkaline styrene-based anion exchange resin, 717-type strongly alkaline styrene-based anion exchange resin, D201-type macroporous and strongly alkaline styrene-based anion exchange resin or D218-type macroporous and strongly alkaline acrylic-based anion exchange resin, D301-G-type macroporous and weakly acidic styrene-based anion exchange resin and D301-type macroporous and weakly alkaline styrene-based anion exchange resin.

Optionally, the amount of the anion resin used and the charged plant raw material had a weight ratio ranging from 1:1 to 1:80 (optionally 1:1 to 1:64, 1:1 to 1:32, 1:1 to 1:24, 1:5 to 1:16, 1:3).

Collection started when liquid flowed out of the anion resin. Optionally, the collection was terminated when the volume of the collected solution reached 0.05 to 10 times the weight of the charged plant raw material (optionally 0.1 to 5 times).

Optionally, the ethanol used for alcohol precipitation and the charged plant raw material had a weight ratio ranging from 1:4 to 1:600 (optionally 1:20 to 1:300, further optionally 1:20 to 1:50, 1:40, 1:80, 1:22). The stirring speed during alcohol precipitation was 10 to 600 rpm (optionally 40 to 500 rpm, 80 to 400 rpm). The alcohol precipitation duration was 12 to 24 h.

Further, prior to alcohol precipitation, the method further includes a step of concentrating by reverse osmosis membrane after impurities were removed from the anion resin effluent by conducting centrifugation or filtration via micro-filtration membrane. The specific gravity of the concentrated liquid might be 1.0 to 1.3, optionally 1.1 to 1.25.

Optionally, the drug further comprises a pharmaceutically acceptable carrier. The carrier is an inactive component that conforms to the administration route or the mode of administration and has no toxic effect on the human body. The carrier may be a solid or liquid excipient. Solid excipients, for example, include microcrystalline cellulose, mannitol, lactose, pregelatinized starch, low-substituted hydroxypropyl cellulose, crospovidone, sodium starch glycolate, aspartame, calcium hydrogen phosphate, sodium lactate, poloxamer, sodium dodecyl sulfate, sodium carboxymethyl cellulose, gelatin, xanthan gum, povidone, starch, magnesium stearate and talc; liquid excipients, for example, include water, ethanol, syrup and glycerol.

In the present disclosure, the mulberry extract is abbreviated as SZ-A.

### Beneficial Effects

Mulberry extract is capable of increasing bone mineral density, promoting osteogenesis of osteoblasts, and inhibiting bone resorption of osteoclasts. Meanwhile, it can also improve muscle content and quality, and facilitate the proliferation and differentiation of myoblasts.

The present disclosure provides use of mulberry extract in preparing drugs or health care products for treating and/or preventing diseases related to abnormal bone metabolism. It can significantly improve bone mineral density, bone calcium content and muscle content in model mice, thereby ultimately ameliorating diseases related to abnormal bone metabolism.

### Description of Drawings

Fig. 1 shows determination results of bone mineral densities, muscle weight percentages, and muscle weight/fat weight ratios of mice in each group in Test Example 1; wherein: *P<0.05, **P<0.01, ***P<0.001 vs. the model group.
Fig. 2 shows pathological section images (2A) of gastrocnemius muscle of mice in each group in Test Example 1, as well as a histogram (2B) of average cross-sectional areas of gastrocnemius muscle fibers, a histogram (2C) of average diameters of gastrocnemius muscle fibers, a histogram (2D) of average perimeters of gastrocnemius muscle fibers, and pathological section images (2E) of tibia in mice.
Fig. 3 shows the micro-CT scanning images of bone tissue of mice in each group in Test Example 2 (3A), together with the histograms of trabecular numbers (3B), trabecular separations (3C), bone mineral densities (3D), bone volume fractions (3E), bone structure model indexes (3F), and trabecular thickness (3G); wherein *P<0.05, **P<0.01 vs. the model group.
Fig. 4 shows determination results of bone mineral densities, muscle weight percentages, and muscle weight/fat weight ratios of mice in each group in Test Example 2; wherein *P<0.05, **P<0.01, ***P<0.001 vs. the model group.
Fig. 5 shows determination results of bone mineral densities of mice in each group in Test Example 2; wherein *P<0.05, **P<0.01 vs. the model group.
Fig. 6 shows the pathological section photographs of femur of mice in each group in Test Example 3.
Fig. 7 shows the effects of mulberry extract on muscle weight percentage in rats with endocrine disorder model induced by LZ+HFD in Test Example 4; wherein: *P<0.05, **P<0.01 vs. the model group.
In Fig. 8, A shows the effect of mulberry extract on pathological sections of gastrocnemius muscle in Apoe mice with diabetic sexual dysfunction model induced by TZ combined with high-fat and high-cholesterol diet in Test Example 5; and B to D are histograms of the effects of mulberry extract on the average areas, average diameters and average perimeters of gastrocnemius muscle fibers in Test Example 5; wherein: *P<0.05, **P<0.01 vs. the model group.
Fig. 9 is a histogram of bone mineral density determination results of mice in each group in Test Example 6; wherein: *P<0.05, **P<0.01, ***P<0.001 vs. the aging group.
Fig. 10 shows histograms of grip strength values of mice in each group in Test Example 7 (*P<0.05 vs. the model group; ###P<0.001 vs. the normal group).
Fig. 11 shows SA-β-galactosidase staining images (A) of the effects of mulberry extracts from different preparation examples on D-galactose-induced senescent C2C12 cells in Test Example 8, and a statistical histogram (B); wherein: ***P<0.001, ****P<0.0001 vs. the model group.
Fig. 12 shows distribution histograms of (A) DNJ, (B) FAG and (C) DAB in bone marrow tissue of rats after intragastric administration of SZ-A (40 mg/kg) in Test Example 9.

### Embodiments

The present disclosure is further illustrated below via specific test examples. It should be understood that the following experiments are only intended to explain the technical solutions of the present disclosure, rather than limiting the protection scope thereof.

The mulberry extract of the present disclosure can be prepared by the following methods:

### I. Preparation Examples of Mulberry Extract

### Preparation Example 1 of Mulberry Extract

1000 kg of fresh Ramulus Mori (Morus serrata Roxb-Yuesang No. 11) was taken and crushed, then added with 4000 L of water and extracted by heating reflux for 2 h; extraction solution was combined, and filtered to remove insoluble substances, and a crude extraction solution is thus afforded. The crude extraction solution was concentrated by heating until a mass percentage content of solid substances was up to 4%, and was kept at 50°C and served as a loading solution for a cation resin column.

150 kg of D113-type macroporous and weakly acidic phenylpropene-based cation resin was used for column packing, and a hydrochloric acid solution of 2 mol/L was used for washing until the pH of an eluent was 4.5; a sodium hydroxide solution of 1 mol/L was used for washing until the pH of an eluent was 8.5; a hydrochloric acid solution of 2 mol/L was used for washing until the pH of an eluent was 4.5; and deionized water, of which the volume was 5 times as that of the column, was used for rinsing, and activation was thus completed. The concentrated extraction solution was loaded, and then was eluted with 1000 L of 2.5 mol/L ammonia water at an elution speed of 6 BV/h; the eluent was collected when pH of the effluent from the cation column was detected to be higher than 7; collection was stopped when the collection solution was up to 900 L, and the collection solution was purified directly through the anion column.

62.5 kg of D218-type macroporous and strongly alkaline acrylic-based anion resin was used for column packing, and a sodium hydroxide solution of 1.5 mol/L was used for washing until the pH of the eluent was 9.0; a hydrochloric acid solution of 1.5 mol/L was used for washing until the pH of the eluent was 3.5; and a sodium hydroxide solution of 1.5 mol/L was used for washing until the pH of the eluent was 9.0, and the activation was thus completed. The collected cation resin eluent was loaded onto the anion resin, and the effluent was collected until the effluent was up to 920 L.

The collection solution was centrifuged to remove impurities, and then was concentrated by means of a reverse osmosis membrane; a specific gravity of concentrated liquid was 1.25; the concentrated liquid was transferred to an alcohol precipitation tank, and 25 L of anhydrous ethanol was added when the stirring paddle was at 500 rpm. After the ethanol was added, stirring was stopped, and alcohol precipitation was performed for 24 h; the supernatant was taken and concentrated under reduced pressure to afford extractum of Ramulus Mori extract (i.e. the mulberry extract SZ-A).

In the extractum of Ramulus Mori extract, alkaloids had a mass percentage content of 52%, polysaccharides had a mass percentage content of 22%, flavonoids had a mass percentage content of 0.8%, and amino acids had a mass percentage content of 20%. Among the alkaloids, 1-DNJ had a content of 60%, FAG had a content of 17%, and DAB had a content of 15%.

### Preparation Example 2 of Mulberry Extract

10 kg of fresh Ramulus Mori (Sangteyou No. 2) were taken and crushed, then were added with 150 L of water in 2 times, and were extracted by means of decocting for 3 h each time; extraction solution was combined, and was filtered to remove insoluble substances. The extraction solution was concentrated by heating until a mass percentage content of solid substances was up to 8%, then was transferred to an alcohol precipitation tank, and was added with 2367.9 g (3 L) of anhydrous ethanol when the stirring paddle was at 300 rpm. After the ethanol was added, stirring was stopped, and alcohol precipitation was performed for 24 h; the supernatant was taken as a loading solution for the cation resin column. 5 kg of 002SC-type strongly acidic styrene-based cation resin was used for column packing, and the cation resin was activated according to the method described in Embodiment 1. The extraction solution was loaded after concentration and alcohol precipitation, and then was eluted with 100 L of 5 mol/L potassium chloride at an elution speed of 5 BV/h; the effluent was detected with 20% silicotungstic acid, and collection was started upon generation of white precipitate; the collection was stopped until the collection solution was up to 25 L; and the collection solution was purified directly through the anion resin column.

10 kg of 711-type strongly alkaline styrene-based anion resin was used for column packing, and the anion resin was activated according to the method described in Preparation Example 1. The collected cation resin eluent was loaded onto the anion resin, and the effluent was collected until the effluent was up to 15 L. The collection solution was reloaded onto the cation resin, and was separated twice by using the cation resin and the anion resin in sequence according to the afore-mentioned method.

The collection solution afforded after three column separations was centrifuged to remove impurities, and then was concentrated through a reverse osmosis membrane, wherein the specific gravity of the concentrated liquid was 1.25; it was transferred to an alcohol precipitation tank, and was added with 125 g of anhydrous ethanol when the stirring paddle was at 1000 rpm. After the ethanol was added, the stirring was stopped, and alcohol precipitation was performed for 24 h; the supernatant was taken and concentrated under reduced pressure to obtain extractum of extract. In addition, fresh Cortex Mori and Folium Mori (Sangteyou No. 2) were taken again and were extracted, wherein the extraction method and parameters were the same as those described above.

In the afforded extract of Ramulus Mori, alkaloids had a mass percentage content of 98%, polysaccharides had a mass percentage content of 0.2%, flavonoids had a mass percentage content of 0.05%, and amino acids had a mass percentage content of 0%. Among alkaloids, 1-DNJ had a content of 99%, FAG had a content of 0.5%, and DAB had a content of 0.4%.

In the afforded mulberry bark extract, alkaloids had a mass percentage content of 95%, polysaccharides had a mass percentage content of 2%, flavonoids had a mass percentage content of 0.1%, and amino acids had a mass percentage content of 1%. Among the alkaloids, 1-DNJ had a content of 96%, FAG had a content of 1.5%, and DAB had a content of 1.4%.

In the afforded mulberry leaf extract, alkaloids had a mass percentage content of 90%, polysaccharides had a mass percentage content of 4%, flavonoids had a mass percentage content of 0.1%, and amino acids had a mass percentage content of 3%. Among the alkaloids, 1-DNJ had a content of 90%, FAG had a content of 3.1%, and DAB had a content of 2.8%.

### Preparation Example 3 of Mulberry Extract

1000 kg of fresh Ramulus Mori (Morus atropurpurea Roxb) was taken and crushed, then was added with 11500 L of water and was extracted by heating reflux for 2 h; extraction solution was combined, and was filtered to remove insoluble substances, and a crude extraction solution is thus afforded. The crude extraction solution was centrifuged to remove impurities, and then was concentrated by means of a reverse osmosis membrane until a percentage of solid substances was up to 1%, and served as a loading solution for a cation resin column.

300 kg of D001-type macroporous and strongly acidic styrene-based cation resin was used for column packing, and the cation resin was activated according to the method described in Preparation 1. The crude extraction solution was loaded after concentration, and then was eluted with 5000 L of 0.04 mol/L ammonium nitrate at an elution speed of 5 BV/h; the effluent was detected with 20% silicotungstic acid, and collection was started upon generation of white precipitate; the collection was stopped until the collection solution was up to 1000 L.

The collection solution afforded after separation of cation column was concentrated by nanofiltration, and then was concentrated under reduced pressure to obtain extractum of extract.

In the afforded Ramulus Mori extract, alkaloids had a mass percentage content of 15%, polysaccharides had a mass percentage content of 20%, flavonoids had a mass percentage content of 7%, and amino acids had a mass percentage content of 45%. Among the alkaloids, 1-DNJ had a content of 55%, FAG had a content of 23%, and DAB had a content of 10%.

### Preparation Example 4 of Mulberry Extract

333 kg of dried Ramulus Mori (Yuesang No. 11) was taken and crushed, then was added with 4000 L of water and was extracted twice by heating reflux, 1 h for each reflux; extraction solution was combined and filtered, and was concentrated until the crude drug quantity reached 1 kg/L.

150 kg of D113-type macroporous and weakly acidic phenylpropene-based cation resin was used for column packing, and a hydrochloric acid solution of 2 mol/L was used for washing until the pH of an eluent was 4.5; a sodium hydroxide solution of 1 mol/L was used for washing until the pH of an eluent was 8.5; a hydrochloric acid solution of 2 mol/L was used for washing until the pH of an eluent was 4.5; and deionized water, of which the volume was 5 times as that of the column, was used for rinsing, and activation was thus completed. The concentrated extraction solution was loaded, and then was eluted with 1000 L of 2.5 mol/L ammonia water at an elution speed of 6 BV/h; the eluent was collected when pH of the effluent from the cation column was detected to be higher than 7; collection was stopped when the collection solution was up to 900 L, and the collection solution was purified directly through the anion column.

125 kg of D218-type macroporous and strongly alkaline acrylic-based anion resin was used for column packing, and a sodium hydroxide solution of 1.5 mol/L was used for washing until the pH of the eluent was 9.0; a hydrochloric acid solution of 1.5 mol/L was used for washing until the pH of the eluent was 3.5; and a sodium hydroxide solution of 1.5 mol/L was used for washing until the pH of the eluent was 9.0, and the activation was thus completed. The collected cation resin eluent was loaded onto the anion resin, and the effluent, of which pH was higher than 8, was collected until the effluent was up to 870 L.

The collection solution afforded after anion column separation was filtered by means of micro-filtration membrane to remove impurities, and then was concentrated through a reverse osmosis membrane, wherein the specific gravity of the concentrated liquid was 1.1; it was transferred to an alcohol precipitation tank, and was added with 15 kg of anhydrous ethanol when the stirring paddle was at 400 rpm. After finishing adding the ethanol, the stirring was stopped, and alcohol precipitation was performed for 24 h; the supernatant was taken and concentrated under reduced pressure to obtain extractum of Ramulus Mori extract. Contents in the sample: alkaloids had a mass percentage content of 80%, polysaccharides had a mass percentage content of 5%, flavonoids had a mass percentage content of 0.1%, and amino acids had a mass percentage content of 4%. Among the alkaloids, 1-DNJ had a content of 75%, FAG had a content of 12%, and DAB had a content of 10%.

### Preparation Example 5 of Mulberry Extract

400 kg of dried Ramulus Mori (Yuesang No. 11) was taken and crushed, then was added with 4000 L of water and was extracted twice by heating reflux, 1 h for each reflux; extraction solution was combined and filtered, and was concentrated until the crude drug quantity reached 1 kg/L.

62.5 kg of D218-type macroporous and strongly alkaline acrylic-based anion resin was used for column packing, and a sodium hydroxide solution of 1.5 mol/L was used for washing until the pH of the eluent was 9.0; a hydrochloric acid solution of 1.5 mol/L was used for washing until the pH of the eluent was 3.5; and a sodium hydroxide solution of 1.5 mol/L was used for washing until the pH of the eluent was 9.0, and the activation was thus completed. The collected extract was concentrated and loaded onto the anion resin, and the effluent was collected.

The collection solution afforded after anion column separation was filtered by means of micro-filtration membrane to remove impurities, then was concentrated through a reverse osmosis membrane, and was further concentrated under reduced pressure and dried to obtain extractum of Ramulus Mori extract. Contents in the sample: alkaloids had a mass percentage content of 3%, polysaccharides had a mass percentage content of 70%, flavonoids had a mass percentage content of 10%, and amino acids had a mass percentage content of 10%. Among the alkaloids, 1-DNJ had a content of 68%, FAG had a content of 17%, and DAB had a content of 8%.

### Preparation Example 6 of Mulberry Extract

1500 kg of fresh Ramulus Mori (Morus serrata Roxb-Yuesang No. 11) was taken and crushed, then was added with 6000 L of water and was extracted by heating reflux for 2 h; extraction solution was combined, and was filtered to remove insoluble substances, and a crude extraction solution is thus afforded. The crude extraction solution was concentrated by heating until a percentage of solid substances was up to 4%, and was kept at 50°C and served as a loading solution for a cation resin column.

100 kg of D113-type macroporous and weakly acidic phenylpropene-based cation resin was used for column packing, and a hydrochloric acid solution of 2 mol/L was used for washing until the pH of an eluent was 4.5; a sodium hydroxide solution of 1 mol/L was used for washing until the pH of an eluent was 8.5; a hydrochloric acid solution of 2 mol/L was used for washing until the pH of an eluent was 4.5; and deionized water, of which the volume was 5 times as that of the column, was used for rinsing, and activation was thus completed. The concentrated extraction solution was loaded, and then was eluted with 1000 L of 2.5 mol/L ammonia water at an elution speed of 6 BV/h; the eluent was collected when pH of the effluent from the cation column was detected to be higher than 7; collection was stopped when the collection solution was up to 900 L, and the collection solution was purified directly through the anion column.

62.5 kg of D218-type macroporous and strongly alkaline acrylic-based anion resin was used for column packing, and a sodium hydroxide solution of 1.5 mol/L was used for washing until the pH of the eluent was 9.0; a hydrochloric acid solution of 1.5 mol/L was used for washing until the pH of the eluent was 3.5; and a sodium hydroxide solution of 1.5 mol/L was used for washing until the pH of the eluent was 9.0, and the activation was thus completed. The collected cation resin eluent was loaded onto the anion resin, and the effluent was collected until the effluent was up to 870 L. The effluent was concentrated under reduced pressure so as to obtain extractum of Ramulus Mori extract, wherein: alkaloids had a mass percentage content of 30%, polysaccharides had a mass percentage content of 35%, flavonoids had a mass percentage content of 2%, and amino acids had a mass percentage content of 25%. Among the alkaloids, 1-DNJ had a content of 62%, FAG had a content of 20%, and DAB had a content of 13%.

### Preparation Example 7 of Mulberry Extract

1000 kg of fresh Ramulus Mori (Morus serrata Roxb-Yuesang No. 11) was taken and crushed, then was added with 4000 L of water and was extracted by heating reflux for 2 h; extraction solution was combined, and was filtered to remove insoluble substances, and a crude extraction solution is thus afforded. The crude extraction solution was concentrated by heating until a percentage of solid substances was up to 4%, and was kept at 50°C and served as a loading solution for a cation resin column.

100 kg of D113-type macroporous and weakly acidic phenylpropene-based cation resin was used for column packing, and a hydrochloric acid solution of 2 mol/L was used for washing until the pH of an eluent was 4.5; a sodium hydroxide solution of 1 mol/L was used for washing until the pH of an eluent was 8.5; a hydrochloric acid solution of 2 mol/L was used for washing until the pH of an eluent was 4.5; and deionized water, of which the volume was 5 times as that of the column, was used for rinsing, and activation was thus completed. The concentrated extraction solution was loaded, and then was eluted with 1000 L of 2.5 mol/L ammonia water at an elution speed of 6 BV/h; the eluent was collected when pH of the effluent from the cation column was detected to be higher than 7; collection was stopped when the collection solution was up to 900 L, and the collection solution was purified directly through the anion column.

62.5 kg of D218-type macroporous and strongly alkaline acrylic-based anion resin was used for column packing, and a sodium hydroxide solution of 1.5 mol/L was used for washing until the pH of the eluent was 9.0; a hydrochloric acid solution of 1.5 mol/L was used for washing until the pH of the eluent was 3.5; and a sodium hydroxide solution of 1.5 mol/L was used for washing until the pH of the eluent was 9.0, and the activation was thus completed. The collected cation resin eluent was loaded onto the anion resin, and the effluent was collected until the effluent was up to 870 L. The effluent was concentrated under reduced pressure so as to obtain extractum of Ramulus Mori extract, wherein: alkaloids had a mass percentage content of 40%, polysaccharides had a mass percentage content of 25%, flavonoids had a mass percentage content of 0.5%, and amino acids had a mass percentage content of 25%. Among the alkaloids, 1-DNJ had a content of 57%, FAG had a content of 24%, and DAB had a content of 16%.

### Preparation Example 8 of Mulberry Extract

333 kg of dried Ramulus Mori (Yuesang No. 11) was taken and crushed, then was added with 4000 L of water and was extracted twice by heating reflux, 1 h for each reflux; extraction solution was combined and filtered, and was concentrated until the crude drug quantity reached 1 kg/L.

150 kg of D113-type macroporous and weakly acidic phenylpropene-based cation resin was used for column packing, and a hydrochloric acid solution of 2 mol/L was used for washing until the pH of an eluent was 4.5; a sodium hydroxide solution of 1 mol/L was used for washing until the pH of an eluent was 8.5; a hydrochloric acid solution of 2 mol/L was used for washing until the pH of an eluent was 4.5; and deionized water, of which the volume was 5 times as that of the column, was used for rinsing, and activation was thus completed. The concentrated extraction solution was loaded, and then was eluted with 1000 L of 2.5 mol/L ammonia water at an elution speed of 6 BV/h; the eluent was collected when pH of the effluent from the cation column was detected to be higher than 7; collection was stopped when the collection solution was up to 900 L, and the collection solution was purified directly through the anion column.

62.5 kg of D218-type macroporous and strongly alkaline acrylic-based anion resin was used for column packing, and a sodium hydroxide solution of 1.5 mol/L was used for washing until the pH of the eluent was 9.0; a hydrochloric acid solution of 1.5 mol/L was used for washing until the pH of the eluent was 3.5; and a sodium hydroxide solution of 1.5 mol/L was used for washing until the pH of the eluent was 9.0, and the activation was thus completed. The collected cation resin eluent was loaded onto the anion resin, and the effluent, of which pH was greater than 8, was collected until the effluent was up to 870 L.

The collection solution afforded after anion column separation was filtered by means of micro-filtration membrane to remove impurities, and then was concentrated through a reverse osmosis membrane, wherein the specific gravity of the concentrated liquid was 1.1; it was transferred to an alcohol precipitation tank, and was added with 15 kg of anhydrous ethanol when the stirring paddle was at 400 rpm. After finishing adding the ethanol, the stirring was stopped, and alcohol precipitation was performed for 24 h; the supernatant was taken and concentrated under reduced pressure to obtain extractum of Ramulus Mori extract. Contents in sample: alkaloids had a mass percentage content of 63%, polysaccharides had a mass percentage content of 23%, flavonoids had a mass percentage content of 1%, and amino acids had a mass percentage content of 5%.

Among the alkaloids, 1-DNJ had a content of 61.9%, FAG had a content of 16.6%, and DAB had a content of 11.1%.

### II. Efficacy Tests of Mulberry Extract

### Test Example 1: High-Fat Diet (HFD) Mice Model

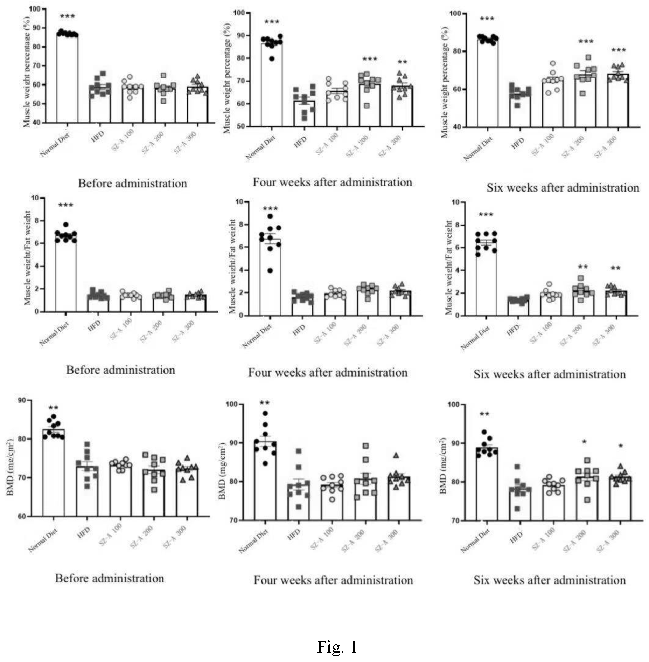

Forty-five healthy male C57 mice aged 6 weeks were randomly divided into a normal control group (NC), a high-fat diet model group (HFD), and mulberry extract (SZ-A) groups with different doses: a low-dose group SZ-A100 (100 mg/kg/d, calculated based on alkaloids), a medium-dose group SZ-A200 (200 mg/kg/d, calculated based on alkaloids), and a high-dose group SZ-A300 (300 mg/kg/d, calculated based on alkaloids), with 9 mice in each group. The mulberry extract used was from Preparation Example 8. Mice in the NC group were fed with basal diet, while those in the HFD model group and SZ-A groups were given high-fat diet (Research Diet, D12492, 60 kcal% Fat); after 17 weeks of feeding, each test group was administered corresponding dose of the test drug continuously for 6 weeks; during the treatment period, high-fat diet feeding was maintained, and body weights, as well as food and water intakes of animals were monitored simultaneously. The model group was given normal saline. Bone mineral density (BMD) and other indicators were detected before administration, and at Week 4 and Week 6 of administration to evaluate the efficacy of SZ-A. Body fat content and muscle content were measured before the experiment, and at Week 4 and Week 6 of treatment. Laboratory mice were anesthetized intramuscularly with Zoletile, placed in a prone position in a FAXITRON ULTRAFOCUS 100 bone densitometer, and scanned by X-ray, so that the body fat content, muscle content and BMD in mice were detected and analyzed. After mice sampling, the tibia and gastrocnemius muscle were collected for HE staining. Femurs were collected, and fixed in 10% neutral buffered formalin for 24 hours, and then redundant soft tissues were removed. The femurs were scanned using a Skyscan 1276 micro-CT with the following settings before images were reconstructed via NRecon software.
(1) The results of bone mineral density, ratio of muscle content to body weight (i.e. muscle weight percentage) and muscle-fat ratio of mice are shown in Fig. 1. According to the results, the bone mineral densities of mice were significantly decreased in the model group; after 6 weeks of treatment, the bone mineral densities of mice were markedly increased in SZ-A groups with all doses, suggesting that mulberry extract could significantly elevate bone mineral density; the muscle weight percentage and muscle-fat ratio were notably reduced in the model group; after 6 weeks of treatment, the muscle weight percentage and muscle-fat ratio of obese mice were remarkably increased in SZ-A groups with all doses.

### (2) Pathology Examination

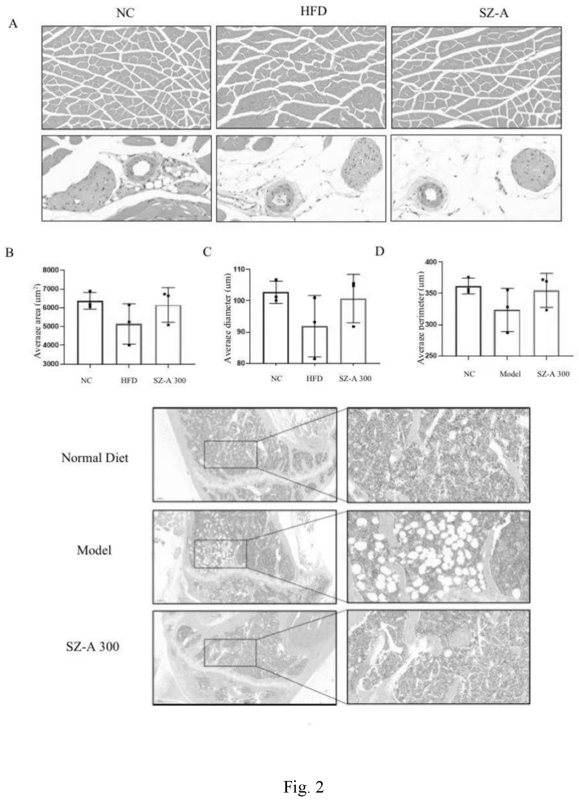

Morphological changes of muscle fibers were observed by HE staining. Fig. 2A shows the pathological sections of mouse gastrocnemius muscle: in the NC group, the skeletal muscle structure was clear, no abnormalities were observed in the gaps of muscle bundles and muscle fibers, and no abnormalities were found in intramuscular nerves and blood vessels. In the HFD model group, a large number of muscle fibers were edematous, and edema and atrophy of muscle fibers coexisted, with edema being the predominant feature; muscle fibers within the same muscle bundle showed significant differences in cross-sectional area and varied in size. Lipid deposition was visible under the intima of arterioles in intramuscular space, with mild thickening of the arterial medial membrane. Decreased density of myelin fibers (demyelination-like changes) in the peripheral intermuscular nerves could be observed. Compared with the model group, SZ-A300 after administration had clear muscle fiber structure, alleviated edema and muscle fiber atrophy, intact arteriole structure without obvious lipid deposition, and relieved peripheral neuropathy.

According to Figs. 2B to 2D, compared with the model group, gastrocnemius muscle fibers in SZ-A300 after administration had significantly increased average cross-sectional area, average diameter and average perimeter.

tibiae were stained with H&E. Pathological results indicated that administration of SZ-A could reduce the number of fat vacuoles in bone tissue, see Fig. 2E.

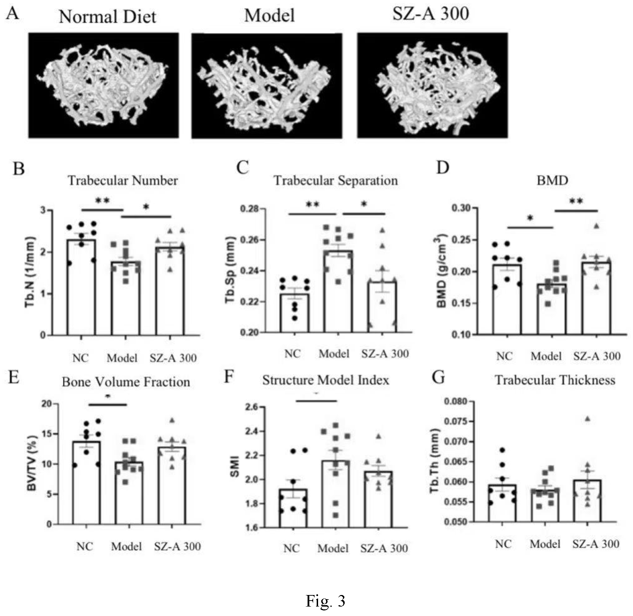

(3) Micro-CT scanning was performed for bone tissue, according to the results shown in Fig. 3, the model group had significantly decreased trabecular number and bone mineral density, as well as increased trabecular separation.; SZ-A300 could improve femoral micro-structure in HFD mice, increased trabecular number and bone mineral density, and reduced trabecular separation; in the model group, bone volume fraction was remarkably decreased and trabecular structure model index was increased, showing that SZ-A tended to elevate bone volume fraction and lower trabecular structure model index.

### Test Example 2: GAN Diet Mice Model

GAN diet is a modified high-fat, high-cholesterol, high-fructose and trans-fat-free diet (containing 40% fat, 20% fructose and 2% cholesterol).

Healthy male C57BL/6J mice aged 6 weeks were selected and divided into a normal control group, a model group and mulberry extract (SZ-A) groups, wherein the mulberry extract groups include a low-dose group SZ-A100 (100 mg/kg/d, calculated based on alkaloids), a medium-dose group SZ-A200 (200 mg/kg/d, calculated based on alkaloids), and a high-dose group SZ-A300 (300 mg/kg/d, calculated based on alkaloids). The mulberry extract used was from Preparation Example 8. Mice in the normal control group were fed with regular diet (normal saline); the model group and SZ-A groups were fed with GAN diet (Research Diet, D09100310) to establish animal models; after 20 weeks of induction, drug administration was performed continuously for 6 weeks, wherein SZ-A groups were given drugs, while the normal control and the model group were given normal saline. Corresponding diet feeding was continued during the treatment period. Details of animal grouping and drug treatment are listed in Table 1 below. Body fat content was detected at Week 4 and Week 6 of administration. Laboratory mice were anesthetized intramuscularly with Zoletile, placed in a prone position in a FAXITRON ULTRAFOCUS 100 bone densitometer, and scanned by X-ray, so that the body fat content, muscle content, fat percentage and BMD were detected and analyzed.

**Table 1 Animal Grouping and Drug Treatment**

| Grouping | Groups | Number of Animals | Administration Frequency | Administration |
|---|---|---|---|---|
| 1 | Normal Control | 8 | Twice a day | Oral |
| 2 | Model Group | 8 | Twice a day | Oral |
| 3 | SZ-A100 Group | 8 | Twice a day | Oral |
| 4 | SZ-A200 Group | 8 | Twice a day | Oral |
| 5 | SZ-A300 Group | 8 | Twice a day | Oral |

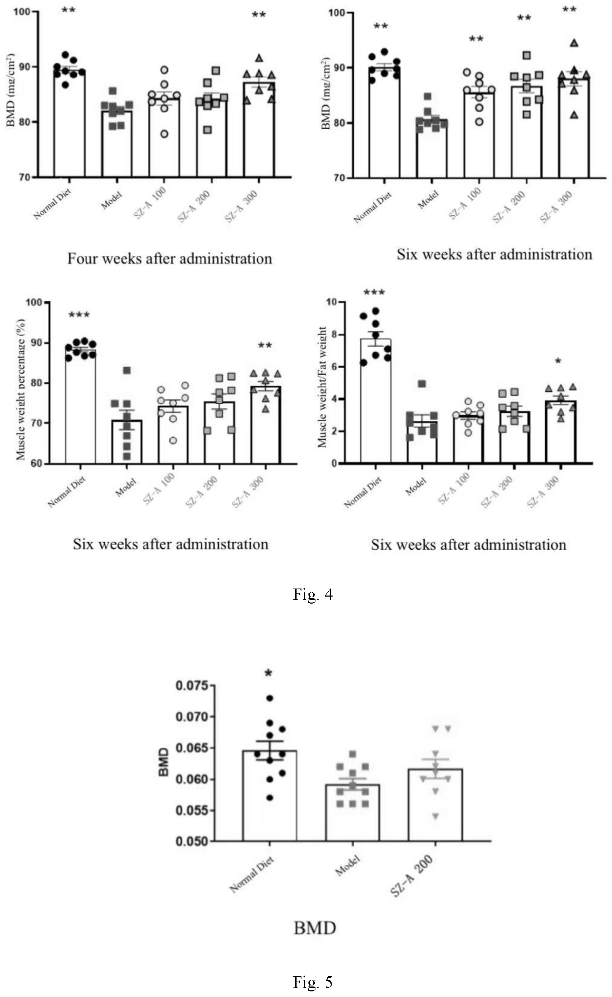

As shown in Fig. 4, the bone mineral density was significantly decreased in the model group; after 6 weeks of treatment, the bone mineral density was remarkably increased in all SZ-A dose groups, indicating that mulberry extract could significantly enhance bone mineral density in mice. After 4 and 6 weeks of treatment, the muscle weight percentage (i.e. the ratio of muscle to body weight) and muscle-fat weight ratio were both significantly elevated.

### Test Example 3: HFD+CCl4 Model

Solution Preparation: Carbon tetrachloride (CCl4) was fully shaken and mixed with olive oil (at a volume ratio of 1:19) to prepare 5% CCl4 solution in a glass bottle.

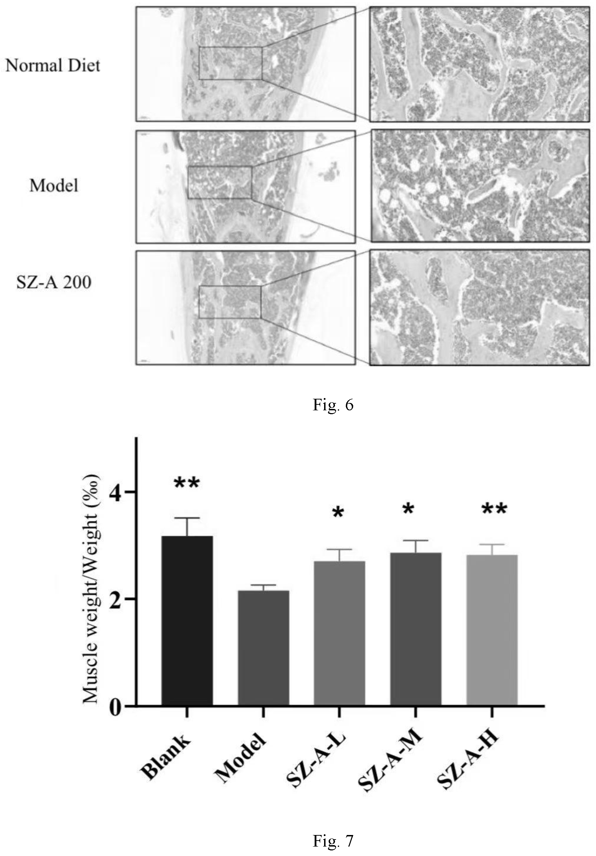

C57BL/6J mice were induced with 60% HFD for 10 weeks, age-matched blank control mice were fed with regular diet for 10 weeks, and then the mice were divided into a normal control group, a model group and an SZ-A administration group, of which the administration dose was 200 mg/kg/d (calculated based on alkaloids). The mulberry extract used was from Preparation Example 8. Mice in the model group and SZ-A group were intraperitoneally injected with 5% CCl4 solution at 2.5 mL/kg body weight twice a week for 4 consecutive weeks, while the normal group was injected with an equal volume of olive oil (e.g., 100 µL for a mouse weighed 40 g). The normal group was intragastrically administered with normal saline, the SZ-A group was treated according to Table 2 below, and the model group was given normal saline. Please refer to Table 2 below for details of grouping and drug treatment. After the experiment, femurs and tibiae of mice were taken, and bone mineral densities were detected using iNSiGHT VET DXA/OT20-2F7113-01 bone densitometer. BMD results of each group are shown in Fig. 5. Femurs were subjected to H&E staining, and the results are shown in Fig. 6.

**Table 2 Experiment Grouping**

| Grouping | Groups | Number of Animals | Administration Frequency | Administration |
|---|---|---|---|---|
| 1 | Normal Control | 10 | Twice a day | Oral |
| 2 | Model Group | 10 | Twice a day | Oral |
| 3 | SZ-A200 Group | 9 | Twice a day | Oral |

As shown in Fig. 5, the bone mineral density was significantly reduced in the model group, while that in the SZ-A200 administration group tended to be increased.

As shown in Fig. 6, the trabecular bone edge of femoral bone tissue in the model group was rough, the trabecular spacing increased, the trabeculae were irregularly arranged with breakpoints, and the number of fat vacuoles in the medullary cavity increased. In the SZ-A200 administration group, the number of trabeculaes increased and the number of fat vacuoles decreased.

### Test Example 4: Pharmacodynamic Test of Mulberry Extract on LZ+HFD-Induced Endocrine Disorder Model

### Mulberry extract from Preparation Example 1

### 1. Drug Preparation

Letrozole (LZ) was prepared to a concentration of 0.25 mg/mL with an administration volume of 0.4 mL/100 g.

1% CMC solution: 1 g of CMC was weighed and added into 100 mL of purified water, and was stirred on a magnetic stirrer at 60°C and 500 r/min until completely dissolved to form a transparent solution.

### 2. Test Grouping

**Table 3**

| Groups | Abbreviation | Number of Animals | Dose (mg/kg/d, calculated based on total Ramulus Mori alkaloids) | Administration | Diet |
|---|---|---|---|---|---|
| Blank Control | Blank | 6 | 0 | | Chow (normal diet) |
| Model Group | Model | 6 | 0 | Intragastric gavage | |
| | SZ-A-L | 6 | 67 | | HFD |
| Administration Group | SZ-A-M | 6 | 100 | | |
| | SZ-A-H | 6 | 150 | | |

### 3. Test Method

Thirty 3-week-old female SD rats with good health and glossy fur were selected and adaptively raised for 3 days, and then were randomly divided into a blank control group (Blank), a model group (Model) and administration groups. Rats in the model and administration groups were fed with high-fat diet HFD (high-fat diet: research diets D12492) for 3 weeks, then intragastrically given letrozole (1 mg/kg/d) for 6 weeks with continuous HFD feeding to establish the rat endocrine disorder model. The drug was administrated to administration groups at doses and in the ways as shown in Table 3; the blank and model groups were given equal volumes of normal saline during administration. After 21 days of treatment, body weights were measured, and then gastrocnemius muscles of the right lower legs were taken and weighed.

According to the results shown in Fig. 7, compared with the model group, the muscle weight percentages (the ratio of muscle to body weight) were significantly increased in all SZ-A dose groups.

### Test Example 5: Apoe Mice Diabetic Sexual Dysfunction Model Induced by STZ Combined with High-Fat High-Cholesterol Diet

Thirty 8-week-old Apoe mice were adaptively fed for 1 week, then were given high-fat high-cholesterol (HFHC) diet for 5 weeks, and were intraperitoneally injected with STZ (streptozotocin, 100 mg/kg/d) once at the sixth week, thereby establishing a mouse model of glucose and lipid metabolism disorder; meanwhile, ten 8-week-old C57 mice were selected and adaptively raised for 1 week, fed with regular diet for 5 weeks, and were intraperitoneally injected with an equal volume of vehicle at the sixth week to form a normal control group.

After the model was successfully established, the model groups were randomly divided into three: a model group, a low-dose (150 mg/kg/d) and a high-dose (300 mg/kg/d) SA-Z (from Preparation Example 8); after drug administration according to Table 4 for 6 continuous weeks, gastrocnemius muscles of mice were collected, fixed in 4% paraformaldehyde solution, embedded in paraffin, and sectioned for H&E staining. Typical visual fields were selected for each sample and photographed under a 200× microscope, so as to measure the average perimeters, average diameters and average cross-sectional areas of muscle fibers.

**Table 4 Animal Grouping and Administration**

| Groups | Abbreviation | Number of Animals | Dose (mg/kg/d, calculated based on total alkaloids) | Administration | Diet |
|---|---|---|---|---|---|
| Blank Control Group | Blank | 10 | 0 | Intragastric administration | Chow |

| Groups | Abbreviation | Number of Animals | Does (mg/kg/d, calculated based on total alkaloids) | Administration | Diet |
|---|---|---|---|---|---|
| Model Group | Model | 10 | 0 | | HFHC |
| SZ-A Group | SZ-A | 10 | 150 | | |

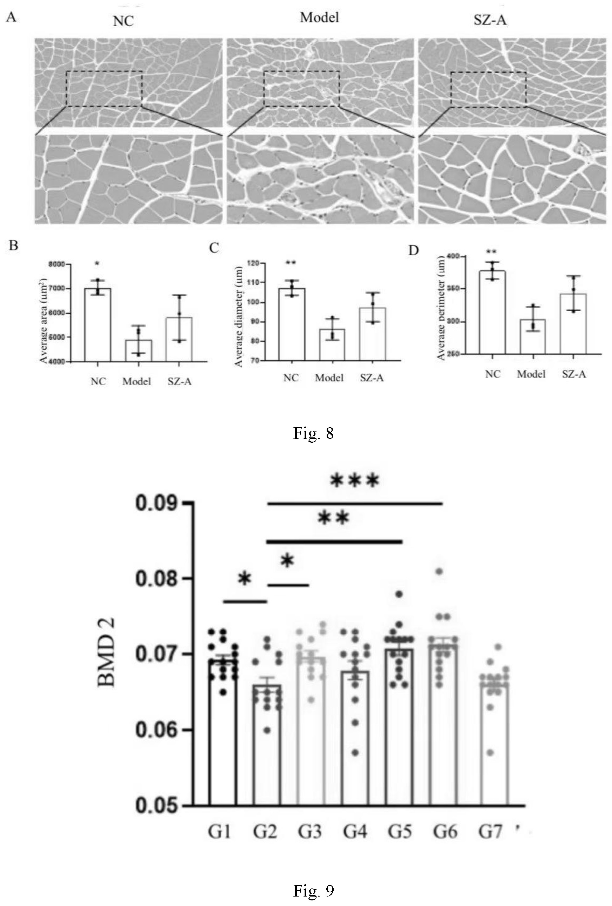

As shown in Fig. 8A, H&E staining results indicated clear structure of the gastrocnemius muscle in the normal group, with no abnormalities observed in the spaces between muscle bundles and muscle fibers. The model group presented coexistence of muscle fiber edema and atrophy, with obvious size differences in cross-sectional areas of muscle fibers within the same bundle. Compared with the model group, the SZ-A group restored clear muscle fiber structure with alleviated edema and atrophy after administration. According to the results shown in Figs. 8B to 8D, average cross-sectional areas, average diameters and average perimeters of gastrocnemius muscle fibers were significantly increased in the SZ-A group relative to the model group.

### Test Example 6: Mice Experiments

### Animal Grouping and Treatment

Fifteen-month-old C57 mice were randomly divided into a natural aging group, an SZ-A administration group and a positive drug group, with 15 mice in each group. The mice were administered intragastrically once daily. Three-month-old C57 mice served as control.

G1 (Normal Control Group): 3-month-old C57 mice, normal diet and water intake, with intragastric administration of distilled water as the control.

G2 (Natural Aging Group): 15-month-old C57 mice, normal diet and water intake, with intragastric administration of distilled water as the control.

G3: 15-month-old C57 mice, normal diet and water intake, given mulberry extract from Preparation Example 1 at 100 mg/kg/d (calculated based on alkaloids) for 5 consecutive months.

G4: 15-month-old C57 mice, normal diet and water intake, given mulberry extract from Preparation Example 1 at 200 mg/kg/d (calculated based on alkaloids) for 5 consecutive months.

G5: 15-month-old C57 mice, normal diet and water intake, given mulberry extract from Preparation Example 7 at 100 mg/kg/d (calculated based on alkaloids) for 5 consecutive months.

G6: 15-month-old C57 mice, normal diet and water intake, given mulberry extract from Preparation Example 7 at 200 mg/kg/d (calculated based on alkaloids) for 5 consecutive months.

G7 (NMN Positive Drug Group): 15-month-old C57 mice, normal diet and water intake, given NMN at 300 mg/kg/d for 5 consecutive months.

After the experiment, mice were anesthetized intramuscularly with Zoletile, and placed in a prone position in a FAXITRON ULTRAFOCUS 100 bone densitometer, so that the bone mineral densities of mice were detected and analyzed

As shown in Fig. 9, BMD was significantly decreased in the aging group compared with the normal control group; compared with the aging group, the bone mineral density increased significantly in the SZ-A group, with a better effect than the NMN group.

### Test Example 7: Normal Diet-Fed db/db Diabetic Mice Model

### Mulberry extract used in this test was from Preparation Example 1.

Sixty 5-week-old male db/db mice were purchased, while twenty db/m male mice served as a normal control group. The mice were housed at room temperature (22°C) with humidity being 60% for 12 hours of light-dark cycle. After 1 week of adaptive feeding with basal diet, 10 µL tail venous blood was collected to measure random blood glucose (PBG), and the mice were weighed. Based on the random blood glucose and body weights, the sixty db/db mice were divided into a model group (db/db), an SZ-A low-dose group (SZ-A100, 100 mg/kg/d, calculated based on alkaloids) and an SZ-A medium-dose group (SZ-A200, 200 mg/kg/d, calculated based on alkaloids), 20 mice in each group; twenty 5-week-old db/m mice served as a normal control group. All mice were fed with basal diet for 2 weeks of adaptation, then were treated with corresponding doses of SZ-A for continuous 8 weeks with sustained basal diet feeding throughout the treatment period; meanwhile, body weights and blood glucose were monitored, and normal saline was provided for the model group and the normal control group. After 8 weeks of administration, a grip tester was used to evaluate the effect of SZ-A on muscle strength in diabetic mice. The grip tester (YUYANBIO) consists of a grab bar and a high-precision force measurement system. Forelimbs of a laboratory mouse were placed on the crossbar of the grab bar, and the mouse tail was gently pulled backward; after the mouse firmly gripped the bar, the tail was pulled backward with steady force until the mouse released its paws; the instrument automatically recorded the maximum grip strength of the animals, and measurement were repeated for several times to ensure reliable results, which were used to indicate muscle strength in mice.

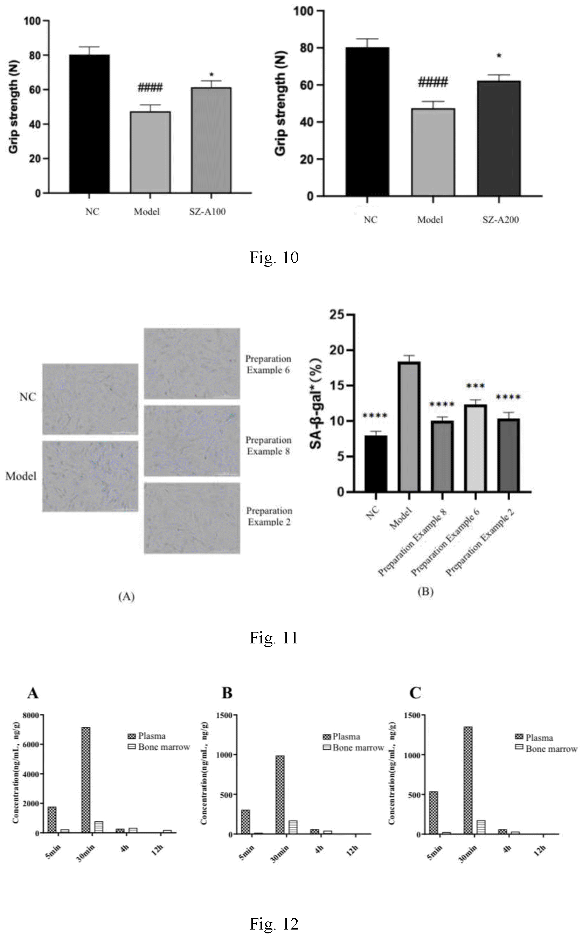

According to the grip strength results of mice shown in Fig. 10, the grip strength was significantly reduced in diabetic mice in the model group; after 8 weeks of treatment, the grip strength of mice was markedly increased in the SZ-A100 mg/kg/d group and the SZ-A200 mg/kg/d group, indicating that mulberry extract could significantly improve muscle strength in db/db diabetic mice.

### Test Example 8:

### Inhibitory Effect of Mulberry Extract on Muscle Cell Injury

C2C12 cells were cultured in growth medium (high-glucose DMEM medium+10% fetal bovine serum) and incubated in a constant temperature incubator at 37°C with 5% CO₂. Cultured to approximately 85%, cells were harvested and divided into a control group (NC), a model group (D-gal) and SZ-A groups (D-gal+SZ-A). Then the culture medium of each group was replaced with differentiation medium (high-glucose DM EM medium+2% horse serum), and, based on the test objective, D-gal and SZ-A (SZ-A from Preparation Examples 2, 6 and 8) were added to the differentiation medium before the end of the experiment. For the model group, D-gal with a concentration of 100 mM was added to the culture medium; and, for SZ-A groups, SZ-A with a concentration of 100 ug/ml and D-gal with a concentration of 100 mM were added to the culture medium with a treatment duration of 48 hours. SA-β-galactosidase staining was used to detect the degree of cell injury in different groups.
Group 1: Control Group
Group 2: Model Group
Group 3: SZ-A Group (SZ-A being Ramulus Mori extract from Preparation Example 2)
Group 4: SZ-A Group (SZ-A being Ramulus Mori extract from Preparation Example 6)
Group 5: SZ-A Group (SZ-A being Ramulus Mori extract from Preparation Example 8)

According to the results in Fig. 11, SZ-A could inhibit C2C12 myocytes injury induced by D-gal.

### Test Example 9: Tissue Distribution of Total Ramulus Mori Alkaloids in Bone Marrow Tissue

### 1. Drugs:

Miglitol Reference Standard (Lot: HRSLW-DM, Content: 100.1%): TCI (Shanghai), Tokyo Chemical Industry Co., Ltd.
DNJ Reference Standard (Lot: 20171024, Content: 99.29%): Beijing Wehand-Bio Pharmaceutical Co., Ltd.
FA Reference Standard (Lot: 16411, Content: 100%): MedChemExpress
DAB Reference Standard (Lot: 0000161756, Content: 100%): Sigma

### 2. Solution Preparation

Preparation of administration solution: 1.65 g of mulberry extract from Preparation Example 8 was precisely weighed, dissolved into an appropriate amount of ultra-pure water, and then shaken well to prepare intragastric administration solution at 4 mg/mL (calculated based on alkaloids).

Preparation of test solution: preparation of 0.1% ammonia water for mobile phase: 4 mL of ammonia water (25%) was accurately transferred, added with 1000 mL of water, shaken well, filtered, and subjected to ultrasonic degassing for 20 minutes.

Preparation of stock solution: 17.0 mg of DNJ Reference Standard, 8.5 mg of FA Reference Standard, 8.5 mg of DAB Reference Standard and 10.0 mg of Miglitol Reference Standard were precisely weighed, respectively dissolved and diluted to volume in a 10 mL measuring flask with Wahaha purified water, and then were shaken well to respectively afford the following stock solutions: DNJ with a concentration of 1.688 mg/mL, FA with a concentration of 0.850 mg/mL, DAB with a concentration of 0.850 mg/mL, and Miglitol with a concentration of 1.0 mg/mL.

### Preparation of mixed reference standard working solution for determination in rat bone marrow tissue:

Appropriate volumes of DNJ, FA and DAB stock solutions were precisely taken, and were mixed and diluted to volume in a 5 mL measuring flask with methanol-water (4:1, v/v) to prepare standard working solutions (DNJ with a concentration of 25000 ng/mL, FA & DAB with a concentration of 12500 ng/mL). Dilutions of the standard working solution were performed to prepare a series of mixed standard curve working solutions with DNJ concentrations of 125, 250, 625, 1250, 2500, 6250 and 12500 ng/ml, and FA and DAB concentrations of 62.5, 125, 312.5, 625, 1250, 3125 and 6250 ng/ml, respectively.

### Preparation of quality control solutions for the determination of rat bone marrow tissue:

Appropriate volumes of DNJ, FA and DAB stock solutions were respectively and precisely taken and mixed into a 5 mL measuring flask so as to be diluted to volume with methanol-water (4:1, v/v) to prepare a standard working solution containing DNJ at 25000 ng/mL and both FA and DAB at 12500 ng/mL. The standard working solution was serially diluted to obtain low, medium and high concentration quality control solutions with DNJ concentrations of 50, 100, 1250 and 10000 ng/mL, and FA and DAB concentrations of 125, 625 and 5000 ng/mL, respectively.

### 3. Animal Administration and Sampling

Twenty-four SD rats (180 to 200 g, half male and half female) aged 6 to 8 weeks were fasted for 12 h with free access to water before administration. Rats were intragastrically given mulberry extract at 40 mg/kg (calculated based on alkaloids), then sacrificed at 5 min, 30 min, 4 h and 12 h post-administration (6 rats per time point, half male and half female). Rat bone marrow (femur) was collected and stored at -80°C.

Processing of bone marrow samples: samples of rat bone marrow tissues were homogenized in a cryogenic grinder, which was pre-cooled to 4°C for 30 min in advance, the homogenization parameters were as follow: frequency 60 Hz, running time 60 s, 2 cycles with 5 s interval. Tissue homogenate was prepared for later use.

50 µL of rat bone marrow tissue homogenate was taken, added with 10 µL of methanol-water solution (4:1, v/v) and 200 µL of protein precipitation solvent containing 100 ng/mL internal standard, subjected to vortex-mix for 2 min, placed at 4°C for 10 min, and was centrifuged at 14000 rpm for 10 min; 100 µL of the supernatant was taken, diluted to 200 µL with 75% acetonitrile (0.1% formic acid), subjected to vortex-shake for 1 min, and was centrifuged at 14000 rpm for 5 min, and then the supernatant was taken for sample injection and determination.

### 4. LC-MS/MS Chromatographic and Mass Spectrometric Conditions

### 4.1 Chromatographic Conditions

Column: XBridgeTM Amide Column (3.5 µm, 4.6 mm×150 mm, Waters, USA); mobile phases: Phase A was water (0.1% ammonia water), and Phase B was acetonitrile; gradient elution: 0.0 to 4.0 min 65% Phase B; 4.0 to 7.0 min 43% Phase B; 7.0 to 13.0 min 65% Phase B; flow rate: 0.5 mL/min; column temperature: 35°C; sampler temperature: 15°C; and injection volume: 5 µL.

### 4.2 Mass Spectrometric Conditions

Electrospray Ionization (ESI) source, Curtain Gas (CUR): 35.0; Collision Gas (CAD): 9; IonSpray Voltage (IS): 5500 V; Temperature (TEM): 500; IonSource Gas1 (GS1): 55.0; IonSource Gas2 (GS2): 50.0; Entrance Potential (EP): 10.0; Collision Cell Exit Potential (CXP): 6.0. The cation scan mode was adopted with multiple reaction monitoring (MRM) as the scanning method. The reaction ions used for quantitative analysis of DNJ, FA, DAB and Miglitol (internal standard, IS) were respectively: m/z 164.3→69.0 (DP 70 ev, CE 27 ev), m/z 148.2→112.1 (DP 56 ev, CE 18 ev), m/z 134.3→68.1 (DP 55 ev, CE 25 ev), and m/z 208.3→146.1 (DP 70 ev, CE 27 ev).

### 5. Result Statistics and Analysis

Drug concentrations in bone marrow tissues were calculated (see Table 4), and the tissue distribution histogram of bone marrow drugs was plotted as Fig. 12.

**Table 4 Drug Concentrations in Rat Bone Marrow Tissues after Intragastric Administration of Ramulus Mori Alkaloids at 40 mg/kg (ng/g, n=6)**

| Marrow Tissue | 5 min | 30 min | 4h | 12 h |
|---|---|---|---|---|
| | Mean + S.D. | Mean + S.D. | Mean + S.D. | Mean + S.D. |
| DNJ | 237.84 + 240.30 | 778.38 + 399.20 | 334.46 + 618.22 | 182.43 + 315.59 |
| FA | 16.22 + 39.72 | 171.62 + 155.66 | 41.89 + 66.70 | 0.00 + 0.00 |
| DAB | 25.00 + 41.22 | 175.00 + 155.36 | 31.76 + 56.08 | 0.00 + 0.00 |

### 6. Results and Discussion

As shown, after intragastric administration of mulberry extract in rats, alkaloids could be detected in bone marrow tissues as early as 5 min post-dosing; DNJ, FA and DAB showed consistent distribution trends in bone marrow tissues, reaching peak concentration at 0.5 h and being almost eliminated from bone marrow tissues at 12 h, indicating that SZ-A had no accumulation risk in bone marrow tissues with good safety profile.

## Claims

1. Use of a mulberry extract in preparing a drug for treating and/or preventing diseases related to abnormal bone metabolism, wherein: taking total weight percentage content of all components in the mulberry extract as 100%, the mulberry extract contains: alkaloids with a weight content of no less than 3% (optionally contains alkaloids with a weight content ranging from 3% to 99%, further optionally contains alkaloids with a weight content ranging from 15% to 99%, further optionally contains alkaloids with a weight content ranging from 30% to 99%, further optionally contains alkaloids with a weight content ranging from 40% to 99%, further optionally contains alkaloids with a weight content ranging from 50% to 99%, and further optionally contains alkaloids with a weight content ranging from 60% to 99%);
and/or polysaccharides with a weight content of no more than 70% (optionally contains polysaccharides with a weight content ranging from 0.2% to 70%, further optionally contains polysaccharides with a weight content ranging from 0.2% to 50%, further optionally contains polysaccharides with a weight content ranging from 0.2% to 35%, further optionally contains polysaccharides with a weight content ranging from 0.2% to 25%, further optionally contains polysaccharides with a weight content ranging from 0.2% to 23%, and further optionally contains polysaccharides with a weight content ranging from 20% to 25%);
and/or flavonoids with a weight content of no more than 10% (optionally contains flavonoids with a weight content ranging from 0.05% to 5%, further optionally contains flavonoids with a weight content ranging from 0% to 2%, further optionally contains flavonoids with a weight content ranging from 0.05% to 2%, further optionally contains flavonoids with a weight content ranging from 0.5% to 1.5%, further optionally contains flavonoids with a weight content ranging from 0% to 1%, and further optionally contains flavonoids with a weight content ranging from 0.05% to 1%);
and/or amino acids with a weight content of no more than 50% (optionally contains amino acids with a weight content ranging from 0% to 30%, further optionally contains amino acids with a weight content ranging from 0% to 25%, further optionally contains amino acids with a weight content ranging from 0% to 20%, further optionally contains amino acids with a weight content ranging from 0% to 5%, further optionally contains amino acids with a weight content ranging from 3% to 25%, and further optionally contains amino acids with a weight content ranging from 5% to 20%);
and/or other components (optionally with a weight content ranging from 0% to 25%, further optionally with a weight content ranging from 0% to 20%, further optionally with a weight content ranging from 0% to 15%, further optionally with a weight content ranging from 0% to 11%, further optionally with a weight content ranging from 2% to 20%, and further optionally with a weight content ranging from 4% to 8%).

2. The use according to claim 1, wherein: the diseases related to abnormal bone metabolism are any one of osteoporosis, sarcopenia, sarcopenia-osteoporosis, muscular atrophy, myasthenia, osteomalacia, rickets, primary hyperparathyroid osteopathy, fluorosis of bone, osteoarthritis and bone dislocation; optionally, the disease related to abnormal bone metabolism is steoporosis, sarcopenia or sarcopenia-osteoporosis.

3. The use according to claim 1 or 2, wherein: the use of the mulberry extract in preparing a drug for treating and/or preventing diseases related to abnormal bone metabolism includes any one of the uses described in the following (b1) to (b3):
(b1) use in preparing a drug for increasing bone mineral density;
(b2) use in preparing a drug for reducing bone marrow lipid vacuoles; and
(b3) use in preparing a drug for improving bone micro-structure;
improvement of bone micro-structure includes at least one of the following: (i) increasing trabecular number; (ii) reducing trabecular separation; (iii) reducing trabecular structure model index; and (iv) increasing bone volume fraction;
and/or the use in any one of the following (1) to (7):
1) use in preparing a drug for increasing muscle content;
2) use in preparing a drug for increasing muscle weight percentage;
3) use in preparing a drug for increasing muscle-fat ratio;
4) use in preparing a drug for inhibiting muscle atrophy and/or edema; optionally, the inhibition of muscle atrophy and/or edema is specifically to increase cross-sectional area of muscle fibers, muscle fiber diameter and/or muscle fiber perimeter;
5) use in preparing a drug for alleviating intermuscular peripheral neuropathy and/or vascular lipid deposition;
6) use in preparing a drug for inhibiting muscle cell injury; and
7) use in preparing a drug for increasing muscle strength.

4. The use according to any one of claims 1 to 3, wherein: taking total weight percentage content of all components in the mulberry extract as 100%, the weight content of each component in the mulberry extract is as follows:
| | |
|---|---|
| alkaloids | 3% to 99%, |
| polysaccharides | 0.2% to 70%, |
| flavonoids | 0% to 10%, |
| amino acids | 0% to 50%, and |
| other components | 0% to 25%; |
optionally, taking total weight percentage content of all components in the mulberry extract as 100%, the weight content of each component in the mulberry extract is as follows:
| | |
|---|---|
| alkaloids | 30% to 99%, |
| polysaccharides | 0.2% to 35%, |
| flavonoids | 0% to 2%, |
| amino acids | 0% to 30%, and |
| other components | 0% to 20%; |
optionally, taking total weight percentage content of all components in the mulberry extract as 100%, the weight content of each component in the mulberry extract is as follows:
| | |
|---|---|
| alkaloids | 30% to 99%, |
| polysaccharides | 0.2% to 35%, |
| flavonoids | 0.05% to 2%, |
| amino acids | 0% to 25%, and |
| other components | 0% to 20%; |
optionally, taking total weight percentage content of all components in the mulberry extract as 100%, the weight content of each component in the mulberry extract is as follows:
| | |
|---|---|
| alkaloids | 40% to 99%, |
| polysaccharides | 0.2% to 25%, |
| flavonoids | 0.05% to 1%, |
| amino acids | 0% to 25%, and |
| other components | 0% to 15%; |
optionally, taking total weight percentage content of all components in the mulberry extract as 100%, the weight content of each component in the mulberry extract is as follows:
| | |
|---|---|
| alkaloids | 50% to 99%, |
| polysaccharides | 0.2% to 23%, |
| flavonoids | 0% to 1%, |
| amino acids | 0% to 20%, and |
| other components | 0% to 11%; |
optionally, taking total weight percentage content of all components in the mulberry extract as 100%, the weight content of each component in the mulberry extract is as follows:
| | |
|---|---|
| alkaloids | 50% to 99%, |
| polysaccharides | 0.2% to 35%, |
| flavonoids | 0% to 2%, |
| amino acids | 0% to 30%, and |
| other components | 0% to 20%; |
optionally, taking total weight percentage content of all components in the mulberry extract as 100%, the weight content of each component in the mulberry extract is as follows:
| | |
|---|---|
| alkaloids | 60% to 99%, |
| polysaccharides | 0.2% to 23%, |
| flavonoids | 0% to 1%, |
| amino acids | 0% to 5%, and |
| other components | 0% to 11%; |
further optionally, taking total weight percentage content of all components in the mulberry extract as 100%, the weight content of each component in the mulberry extract is as follows:
| | |
|---|---|
| alkaloids | 50% to 65%, |
| polysaccharides | 20% to 25%, |
| flavonoids | 0.5% to 1.5%, |
| amino acids | 3% to 25%, and |
| other components | 2% to 20%; |
further optionally, taking total weight percentage content of all components in the mulberry extract as 100%, the weight content of each component in the mulberry extract is as follows:
| | |
|---|---|
| alkaloids | 50% to 65%, |
| polysaccharides | 20% to 25%, |
| flavonoids | 0.5% to 1.5%, |
| amino acids | 5% to 20%, and |
| other components | 4% to 8%; and |
further optionally, taking total weight percentage content of all components in the mulberry extract as 100%, the weight content of each component in the mulberry extract is as follows:
| | |
|---|---|
| alkaloids | 15% to 99%, |
| polysaccharides | 0.2% to 20%, |
| flavonoids | 0% to 10%, |
| amino acids | 0% to 45%, and |
| other components | 0% to 15%. |

5. The use according to any one of claims 1 to 4, wherein: the alkaloids include one or more of 1-deoxynojirimycin (DNJ), N-methyl-1-deoxynojirimycin, fagomine (FAG), 3-epi-fagomine, 1,4-dideoxy-1,4-imino-D-arabinitol (DAB), calysteginB2, calysteginC1, 2-O-(α-D-galactopyranosyl)-1-deoxynojirimycin, 6-O-(β-D-glucopyranosyl)-1-deoxynojirimycin, and 1,4-dideoxy-1,4-imino-(2-O-β-D-glucopyranosyl)-D-arabinitol; and
weight percentage of DNJ is no less than 50% (optionally 60% to 99%) of total alkaloids.

6. The use according to any one of claims 1 to 5, wherein:
the preparation of the mulberry extract includes the following steps of:
1) preparing a crude extraction solution of Moraceae plants; and
2) separating the crude extraction solution via a cation resin and/or an optional anion resin to obtain a resin effluent.

7. The use according to claim 6, wherein: the method further includes the following steps of:
3) subjecting the resin effluent afforded in step 2) to alcohol precipitation and collecting the supernatant; and
4) concentrating and/or drying the supernatant;
optionally, the method further includes the following step of: concentrating and/or drying the resin effluent afforded in step 2).

8. The use according to any one of claims 1 to 7, wherein: the mulberry extract acts on human beings or mammals.

9. The use according to any one of claims 1 to 8, wherein: the drug is in an oral dosage form; optionally, the drug is a tablet, a capsule, an oral solution, an oral emulsion, a pill, a granule, a syrup and a powder.
